(19) Europäisches Patentamt / European Patent Office / Office européen des brevets

(11) EP 2 751 131 B1

(12) **EUROPEAN PATENT SPECIFICATION**

(45) Date of publication and mention of the grant of the patent:
**22.03.2017 Bulletin 2017/12**

(21) Application number: **12836934.5**

(22) Date of filing: **29.09.2012**

(51) Int Cl.:
***C07K 14/385*** *(2006.01)* ***C12N 9/30*** *(2006.01)*
***C12N 15/56*** *(2006.01)* ***C12N 15/63*** *(2006.01)*
***C12P 19/14*** *(2006.01)*

(86) International application number:
**PCT/CN2012/082436**

(87) International publication number:
**WO 2013/044867 (04.04.2013 Gazette 2013/14)**

# (54) POLYPEPTIDES HAVING ALPHA-AMYLASE ACTIVITY AND POLYNUCLEOTIDES ENCODING SAME

POLYPEPTIDE MIT ALPHA-AMYLASE-AKTIVITÄT UND DAFÜR KODIERENDE POLYNUKLEOTIDE

POLYPEPTIDES À ACTIVITÉ ALPHA-AMYLASE ET POLYNUCLÉOTIDES CODANT POUR CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2011 PCT/CN2011/080465**

(43) Date of publication of application:
**09.07.2014 Bulletin 2014/28**

(73) Proprietors:
- **Novozymes A/S**
  **2880 Bagsvaerd (DK)**
- **Novozymes North America, Inc.**
  **Franklinton, North Carolina 27525 (US)**

(72) Inventors:
- **LI, Ming**
  **Chaoyang District,**
  **Beijing 100101 (CN)**
- **DUAN, Junxin**
  **Haidian District**
  **Beijing 100089 (CN)**
- **TSUTSUMI, Noriko**
  **Ichikawa-shi City 272-0823 (JP)**
- **COWARD-KELLY, Guillermo**
  **Wake Forest, NC 27587 (US)**
- **LUNDKVIST, Henrik**
  **SE-217 49 Malmö (SE)**

(74) Representative: **Rasmussen, Preben**
**Novozymes A/S**
**Patents**
**Krogshoejvej 36**
**2880 Bagsvaerd (DK)**

(56) References cited:
**WO-A1-2006/066579** **WO-A1-2006/089107**
**WO-A1-2008/046855** **WO-A2-03/012071**
**WO-A2-03/016535** **WO-A2-03/016535**
**WO-A2-2006/069290**

- **DATABASE UniProt [Online] 16 December 2008 (2008-12-16), "SubName: Full=Pc16g00630 protein {ECO:0000313|EMBL:CAP92733.1}; Flags: Precursor;", XP002733591, retrieved from EBI accession no. UNIPROT:B6H6W6 Database accession no. B6H6W6**
- **DATABASE UniProt [Online] 3 March 2009 (2009-03-03), "SubName: Full=Alpha-amylase, putative {ECO:0000313|EMBL:EED55077.1};", XP002733592, retrieved from EBI accession no. UNIPROT:B8N2R4 Database accession no. B8N2R4**
- **DATABASE GENBANK [Online] 14 August 2009 XP003032859 Database accession no. XP_002560482**
- **DATABASE GENBANK [Online] 14 August 2009 XP003032857 Database accession no. XP_002560950**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 751 131 B1

SEQUENCE LISTING

<110> Novozymes A/S Novozymes North America, Inc. Li, Ming Duan, Junxin Tsutsumi, Noriko Coward-Kelly, Guillermo Lundkvist, Henrik

<120> POLYPEPTIDES HAVING ALPHA-AMYLASE ACTIVITY AND POLYNUCLEOTIDES ENCODING SAME

<130> 12277-WO-PCT[2]

<160> 8

<170> PatentIn version 3.5

<210> 1
<211> 1587
<212> DNA
<213> Penicillium oxalicum

<220>
<221> sig-peptide
<222> (1)..(75)

<220>
<221> exon
<222> (1)..(1011)

<220>
<221> exon
<222> (1081)..(1584)

<400> 1

```
atg ttc ttc aca tcc tcg gtc tgc tgg gcg gca ggc ctc acc acc ctg        48
Met Phe Phe Thr Ser Ser Val Cys Trp Ala Ala Gly Leu Thr Thr Leu
1               5                   10                  15

tca tgc ctg ctg aac ccg gtt ctc gcc gcg gac atg gct gca tgg aag        96
Ser Cys Leu Leu Asn Pro Val Leu Ala Ala Asp Met Ala Ala Trp Lys
            20                  25                  30

act cga tcc gtg tac caa acc atg acc gat cgg ttc gct cgc ccg gac       144
Thr Arg Ser Val Tyr Gln Thr Met Thr Asp Arg Phe Ala Arg Pro Asp
        35                  40                  45

ggg tcg atg acg gct ccg tgc aac gcc tcc gcg ggc ctt tac tgc gga       192
Gly Ser Met Thr Ala Pro Cys Asn Ala Ser Ala Gly Leu Tyr Cys Gly
    50                  55                  60

gga acg tgg aag ggg aca atg aat aag ttg gat tat att cag gac atg       240
Gly Thr Trp Lys Gly Thr Met Asn Lys Leu Asp Tyr Ile Gln Asp Met
65                  70                  75                  80

gga ttc gac gcg atc atg atc tcc ccg atc gta aag aac atc gag ggt       288
Gly Phe Asp Ala Ile Met Ile Ser Pro Ile Val Lys Asn Ile Glu Gly
                85                  90                  95

cgg gtc tgg tat ggt gaa gcc tat cac ggg tac tgg cca cag gat atg       336
```

```
Arg Val Trp Tyr Gly Glu Ala Tyr His Gly Tyr Trp Pro Gln Asp Met
            100                 105                 110

tac caa ctc aac cct cac ttt ggc acc gag caa gag ttg cat gat ctc      384
Tyr Gln Leu Asn Pro His Phe Gly Thr Glu Gln Glu Leu His Asp Leu
        115                 120                 125

gtc gat gcc att cat gcg cgc ggc atg tat atc ctc ttg gac agc gtc      432
Val Asp Ala Ile His Ala Arg Gly Met Tyr Ile Leu Leu Asp Ser Val
    130                 135                 140

att aac aac atg gcc tgg atg acc cgc ggc cag aac ccg gcc acg cac      480
Ile Asn Asn Met Ala Trp Met Thr Arg Gly Gln Asn Pro Ala Thr His
145                 150                 155                 160

atc gac tat tcc gcc ttg acc ccg ttc aac aac cag cag tac tat cac      528
Ile Asp Tyr Ser Ala Leu Thr Pro Phe Asn Asn Gln Gln Tyr Tyr His
                165                 170                 175

cca tac tgc aag atc aag aat tgg aac aac tac acg gat gcc caa ctg      576
Pro Tyr Cys Lys Ile Lys Asn Trp Asn Asn Tyr Thr Asp Ala Gln Leu
            180                 185                 190

tgc caa aca gga gac gac cag gtc gcc ttg ccc gat ctg ttt acc gaa      624
Cys Gln Thr Gly Asp Asp Gln Val Ala Leu Pro Asp Leu Phe Thr Glu
        195                 200                 205

cat gag gac gtt cag aag aca ttg gag gac tgg gca acc gac gtg atc      672
His Glu Asp Val Gln Lys Thr Leu Glu Asp Trp Ala Thr Asp Val Ile
    210                 215                 220

aag aaa tat aac atc gac ggc ctt cga ctc gat gcg gtc aag agt ttg      720
Lys Lys Tyr Asn Ile Asp Gly Leu Arg Leu Asp Ala Val Lys Ser Leu
225                 230                 235                 240

aca ccg agc ttc ctg gcc aag ttt gtc aag aac gtc gga ggc ttc atg      768
Thr Pro Ser Phe Leu Ala Lys Phe Val Lys Asn Val Gly Gly Phe Met
                245                 250                 255

acc gga gaa cag ttc gaa aga gac tcc aag atc atc tgc gac tgg caa      816
Thr Gly Glu Gln Phe Glu Arg Asp Ser Lys Ile Ile Cys Asp Trp Gln
            260                 265                 270

aag aac tat atc tcc agc atg ccc aac tac ccc atg tac tat tcc atg      864
Lys Asn Tyr Ile Ser Ser Met Pro Asn Tyr Pro Met Tyr Tyr Ser Met
        275                 280                 285

gtc gag gcc ttt acc cag gga aac atg tca gat ctc gct gtc caa att      912
Val Glu Ala Phe Thr Gln Gly Asn Met Ser Asp Leu Ala Val Gln Ile
    290                 295                 300

gag acg atg aag tcg ctt tgt gcg gac gtg aca gag atg gtc tcc ttc      960
Glu Thr Met Lys Ser Leu Cys Ala Asp Val Thr Glu Met Val Ser Phe
305                 310                 315                 320

tca gaa aat cac gat att gct cgc gtt cgt gcg ctg agg gac gat ctc     1008
Ser Glu Asn His Asp Ile Ala Arg Val Arg Ala Leu Arg Asp Asp Leu
                325                 330                 335

tcg gtatgtataa tcctatggtc cccagcagtg aatgatcact gaccgagtct          1061
Ser
```

```
ctctcttccc ccgacgcag att gcc aaa acc ttc ctc acg ttc acg tta ctg      1113
                     Ile Ala Lys Thr Phe Leu Thr Phe Thr Leu Leu
                             340                     345

ttc gac gga atc ccc atg ctc tat cag ggc caa gaa cag ttc ttg gac      1161
Phe Asp Gly Ile Pro Met Leu Tyr Gln Gly Gln Glu Gln Phe Leu Asp
    350                 355                 360

ggg ttg tct agt ccc gag aat cgt caa gcg atc tgg ctc acg ggc tac      1209
Gly Leu Ser Ser Pro Glu Asn Arg Gln Ala Ile Trp Leu Thr Gly Tyr
365                 370                 375                 380

aac tcc aac gcg ccc ctc tac cag ctc acc aag gcc ctc aac agc ctt      1257
Asn Ser Asn Ala Pro Leu Tyr Gln Leu Thr Lys Ala Leu Asn Ser Leu
                385                 390                 395

cgg cgc cat gcc ctt gag ctg gat ccc aac tac gtc aac atc cca acc      1305
Arg Arg His Ala Leu Glu Leu Asp Pro Asn Tyr Val Asn Ile Pro Thr
            400                 405                 410

tac ccc atc tat cgg ggt gcc agt gag att gcg gtc agt aag ggt gtg      1353
Tyr Pro Ile Tyr Arg Gly Ala Ser Glu Ile Ala Val Ser Lys Gly Val
        415                 420                 425

cag ggt cga cag gtc gtc acc gtc gtc acc aat caa ggc gtc aag ggt      1401
Gln Gly Arg Gln Val Val Thr Val Val Thr Asn Gln Gly Val Lys Gly
    430                 435                 440

ggc tcc tac acg ctc acc ttg ccc gcg tcc ttc aac gcg atg acg ccc      1449
Gly Ser Tyr Thr Leu Thr Leu Pro Ala Ser Phe Asn Ala Met Thr Pro
445                 450                 455                 460

gtg acg gag atc atc acc tgt tcg aat tac acg gtc gat gcc cgg gga      1497
Val Thr Glu Ile Ile Thr Cys Ser Asn Tyr Thr Val Asp Ala Arg Gly
                465                 470                 475

tcg ctg gtc gtg cag atg gac aaa gga gag cct cgg gtc ttc ttc ccg      1545
Ser Leu Val Val Gln Met Asp Lys Gly Glu Pro Arg Val Phe Phe Pro
            480                 485                 490

aca cag ttg atg gaa ggc agc gga ttg tgt ggt tat tcc taa              1587
Thr Gln Leu Met Glu Gly Ser Gly Leu Cys Gly Tyr Ser
        495                 500                 505
```

<210> 2
<211> 505
<212> PRT
<213> Penicillium oxalicum

<400> 2

```
Met Phe Phe Thr Ser Ser Val Cys Trp Ala Ala Gly Leu Thr Thr Leu
1               5                   10                  15

Ser Cys Leu Leu Asn Pro Val Leu Ala Ala Asp Met Ala Ala Trp Lys
            20                  25                  30

Thr Arg Ser Val Tyr Gln Thr Met Thr Asp Arg Phe Ala Arg Pro Asp
        35                  40                  45
```

```
Gly Ser Met Thr Ala Pro Cys Asn Ala Ser Ala Gly Leu Tyr Cys Gly
    50                  55                  60

Gly Thr Trp Lys Gly Thr Met Asn Lys Leu Asp Tyr Ile Gln Asp Met
65                  70                  75                  80

Gly Phe Asp Ala Ile Met Ile Ser Pro Ile Val Lys Asn Ile Glu Gly
                85                  90                  95

Arg Val Trp Tyr Gly Glu Ala Tyr His Gly Tyr Trp Pro Gln Asp Met
            100                 105                 110

Tyr Gln Leu Asn Pro His Phe Gly Thr Glu Gln Glu Leu His Asp Leu
        115                 120                 125

Val Asp Ala Ile His Ala Arg Gly Met Tyr Ile Leu Leu Asp Ser Val
    130                 135                 140

Ile Asn Asn Met Ala Trp Met Thr Arg Gly Gln Asn Pro Ala Thr His
145                 150                 155                 160

Ile Asp Tyr Ser Ala Leu Thr Pro Phe Asn Asn Gln Gln Tyr Tyr His
                165                 170                 175

Pro Tyr Cys Lys Ile Lys Asn Trp Asn Asn Tyr Thr Asp Ala Gln Leu
            180                 185                 190

Cys Gln Thr Gly Asp Asp Gln Val Ala Leu Pro Asp Leu Phe Thr Glu
        195                 200                 205

His Glu Asp Val Gln Lys Thr Leu Glu Asp Trp Ala Thr Asp Val Ile
    210                 215                 220

Lys Lys Tyr Asn Ile Asp Gly Leu Arg Leu Asp Ala Val Lys Ser Leu
225                 230                 235                 240

Thr Pro Ser Phe Leu Ala Lys Phe Val Lys Asn Val Gly Gly Phe Met
                245                 250                 255

Thr Gly Glu Gln Phe Glu Arg Asp Ser Lys Ile Ile Cys Asp Trp Gln
            260                 265                 270

Lys Asn Tyr Ile Ser Ser Met Pro Asn Tyr Pro Met Tyr Tyr Ser Met
        275                 280                 285

Val Glu Ala Phe Thr Gln Gly Asn Met Ser Asp Leu Ala Val Gln Ile
    290                 295                 300
```

```
Glu Thr Met Lys Ser Leu Cys Ala Asp Val Thr Glu Met Val Ser Phe
305                 310                 315                 320

Ser Glu Asn His Asp Ile Ala Arg Val Arg Ala Leu Arg Asp Asp Leu
                325                 330                 335

Ser Ile Ala Lys Thr Phe Leu Thr Phe Thr Leu Leu Phe Asp Gly Ile
            340                 345                 350

Pro Met Leu Tyr Gln Gly Gln Glu Gln Phe Leu Asp Gly Leu Ser Ser
        355                 360                 365

Pro Glu Asn Arg Gln Ala Ile Trp Leu Thr Gly Tyr Asn Ser Asn Ala
    370                 375                 380

Pro Leu Tyr Gln Leu Thr Lys Ala Leu Asn Ser Leu Arg Arg His Ala
385                 390                 395                 400

Leu Glu Leu Asp Pro Asn Tyr Val Asn Ile Pro Thr Tyr Pro Ile Tyr
                405                 410                 415

Arg Gly Ala Ser Glu Ile Ala Val Ser Lys Gly Val Gln Gly Arg Gln
            420                 425                 430

Val Val Thr Val Val Thr Asn Gln Gly Val Lys Gly Gly Ser Tyr Thr
        435                 440                 445

Leu Thr Leu Pro Ala Ser Phe Asn Ala Met Thr Pro Val Thr Glu Ile
    450                 455                 460

Ile Thr Cys Ser Asn Tyr Thr Val Asp Ala Arg Gly Ser Leu Val Val
465                 470                 475                 480

Gln Met Asp Lys Gly Glu Pro Arg Val Phe Phe Pro Thr Gln Leu Met
                485                 490                 495

Glu Gly Ser Gly Leu Cys Gly Tyr Ser
            500                 505
```

<210> 3
<211> 2354
<212> DNA
<213> Penicillium oxalicum

<220>
<221> six-peptide
<222> (1)..(60)

<220>

<221> exon
<222> (1)..(156)

<220>
<221> exon
<222> (235)..(273)

<220>
<221> exon
<222> (335)..(450)

<220>
<221> exon
<222> (503)..(611)

<220>
<221> exon
<222> (671)..(899)

<220>
<221> exon
<222> (962)..(1124)

<220>
<221> exon
<222> (1173)..(1319)

<220>
<221> exon
<222> (1375)..(1615)

<220>
<221> exon
<222> (1695)..(2354)

<400> 3

```
atg aaa ttc ctt gga cta gct gct ttg ttt ctt gcc cag acc gtg gcg        48
Met Lys Phe Leu Gly Leu Ala Ala Leu Phe Leu Ala Gln Thr Val Ala
1               5                   10                  15

ggt ctg acg gct gcc caa tgg cgc agc caa tcg atc tac ttc ctc atg        96
Gly Leu Thr Ala Ala Gln Trp Arg Ser Gln Ser Ile Tyr Phe Leu Met
            20                  25                  30

act gat cgg ttt ggt cga acc gac aag tcc gtg act gcc cca tgc aac       144
Thr Asp Arg Phe Gly Arg Thr Asp Lys Ser Val Thr Ala Pro Cys Asn
        35                  40                  45

aca aat gac cga gtaagttctc ccccctttttt cgcgtcgggg ggtggttcaa          196
Thr Asn Asp Arg
    50

aacttttgtc gtctgtggta ctgattgaat tctccaag gta tac tgt ggt gga act     252
                                          Val Tyr Cys Gly Gly Thr
                                                  55

tgg caa ggc att atc aat caa gtgagatgca tccgcagccc atggataagg          303
Trp Gln Gly Ile Ile Asn Gln
    60                  65

taaacaatct aggttctgac gtagcttcaa g ttg gat tac atc caa gga atg        355
```

```
                                      Leu Asp Tyr Ile Gln Gly Met
                                                      70

gga ttt act gcg att tgg atc act cca gtc aca gag cag ctc cct caa        403
Gly Phe Thr Ala Ile Trp Ile Thr Pro Val Thr Glu Gln Leu Pro Gln
        75                  80                  85

gat act ggg gac ggt gaa gca tac cac gga tat tgg caa cag gaa at         450
Asp Thr Gly Asp Gly Glu Ala Tyr His Gly Tyr Trp Gln Gln Glu Ile
    90                  95                  100

gtatatgcat cttccaactt cgccccaaat cctctctgac aaggcgatac ag a tac        506
                                                            Tyr
                                                            105

aat gtc aac aac aac tac ggc act gct gcc gat ctc aag gct ctt tcg        554
Asn Val Asn Asn Asn Tyr Gly Thr Ala Ala Asp Leu Lys Ala Leu Ser
                110                 115                 120

caa gcc ctt cac agt cgt gga atg tac ctc atg gtt gat gtt gtc gcg        602
Gln Ala Leu His Ser Arg Gly Met Tyr Leu Met Val Asp Val Val Ala
            125                 130                 135

aac cac atg gtgacttggc tctctctacg ggtgaaaacg agcaggcttc                651
Asn His Met
        140

taactttttc ggtctccag ggc tac gcg ggg gcc gga aac acc gtc gac tac       703
                     Gly Tyr Ala Gly Ala Gly Asn Thr Val Asp Tyr
                                     145                 150

agt gtg ttc aag cca ttc agc tcc tct tcg tac ttc cac ccg tat tgt        751
Ser Val Phe Lys Pro Phe Ser Ser Ser Ser Tyr Phe His Pro Tyr Cys
            155                 160                 165

ttg atc agc gat tac tca aat cag aca aat gtc gag gac tgt tgg ctt        799
Leu Ile Ser Asp Tyr Ser Asn Gln Thr Asn Val Glu Asp Cys Trp Leu
        170                 175                 180

ggg gac acc acc gtc tca ctc ccc gat ctt gat acc act ctt tct tcc        847
Gly Asp Thr Thr Val Ser Leu Pro Asp Leu Asp Thr Thr Leu Ser Ser
    185                 190                 195

gtg cag acg atc tgg tat aat tgg gtc agt gac cta gtt tca aac tat        895
Val Gln Thr Ile Trp Tyr Asn Trp Val Ser Asp Leu Val Ser Asn Tyr
200                 205                 210                 215

tcc a gtgagtatga cctgaaaaga ggccgtgaat tcattcagca aagcctgaca           949
Ser

ttctgtaact ag tc  gac ggt ctt cgg atc gat act gtc aag cac gtt caa      999
                Ile Asp Gly Leu Arg Ile Asp Thr Val Lys His Val Gln
                            220                 225

aag tca ttc tgg cct ggc tat cag agt gct gct ggt gtc tac tgt gtt       1047
Lys Ser Phe Trp Pro Gly Tyr Gln Ser Ala Ala Gly Val Tyr Cys Val
230                 235                 240                 245

gga gag gtc ttc agt ggt gat ccg gct tac act tgc cct tat caa aac       1095
Gly Glu Val Phe Ser Gly Asp Pro Ala Tyr Thr Cys Pro Tyr Gln Asn
                250                 255                 260
```

```
tac ctt gat ggg gtt ttg aat tat cca at  gtaagccgca tctcatcaat        1144
Tyr Leu Asp Gly Val Leu Asn Tyr Pro Ile
            265                 270

tcgctggata caatctgacc agacgcag c tat tac caa tta ctc ggc gca ttt     1197
                                 Tyr Tyr Gln Leu Leu Gly Ala Phe
                                             275

aaa tca acc agt gga agc atc agc agc ctt tac aac atg atc aac tct      1245
Lys Ser Thr Ser Gly Ser Ile Ser Ser Leu Tyr Asn Met Ile Asn Ser
280                 285                 290                 295

gtt gcg tcc gac tgt gct gac cca acc ctg ctg ggt aac ttt att gaa      1293
Val Ala Ser Asp Cys Ala Asp Pro Thr Leu Leu Gly Asn Phe Ile Glu
                300                 305                 310

aac cat gat aac cca cgc ttt gct tc  gtaagttggg atttttgcct            1339
Asn His Asp Asn Pro Arg Phe Ala Ser
            315

tgcgtgtctg aactgagctc atccgcaaaa tgaag g tac aca agc gac tac tcg     1393
                                         Tyr Thr Ser Asp Tyr Ser
                                                         325

caa gcg aaa aat gtc atc tcg ttc att ttc ttg tct gat ggc att ccg      1441
Gln Ala Lys Asn Val Ile Ser Phe Ile Phe Leu Ser Asp Gly Ile Pro
            330                 335                 340

atc gtc tat gcc gga cag gaa cag cac tac agt ggc ggt aac gac cct      1489
Ile Val Tyr Ala Gly Gln Glu Gln His Tyr Ser Gly Gly Asn Asp Pro
        345                 350                 355

gct aac cgt gaa gcg act tgg ctt tcc gga tac tca aag aac gct caa      1537
Ala Asn Arg Glu Ala Thr Trp Leu Ser Gly Tyr Ser Lys Asn Ala Gln
    360                 365                 370

ctg tac caa cac att gct tcg acg aac aaa atc cgc agt ctt gcg att      1585
Leu Tyr Gln His Ile Ala Ser Thr Asn Lys Ile Arg Ser Leu Ala Ile
375                 380                 385                 390

tca aag gat gcc aac tac atc act tcc aag gtgtgtgaat ggagtgcaac        1635
Ser Lys Asp Ala Asn Tyr Ile Thr Ser Lys
                395                 400

ttctcttttc ttgcaaagag aaaataaagg gctggaaaaa gctaatttga cggacatag     1694

aac aat gct ttc tac act gac agt aac acc atc gcc atg aag aaa gga      1742
Asn Asn Ala Phe Tyr Thr Asp Ser Asn Thr Ile Ala Met Lys Lys Gly
                405                 410                 415

tcc tcc gga tcg cag gtc gtg aca gtt ctc tca aac cgt ggc tcg tca      1790
Ser Ser Gly Ser Gln Val Val Thr Val Leu Ser Asn Arg Gly Ser Ser
            420                 425                 430

ggt agc tcg tac act ttg agt ctc agc ggc agc ggc tat gcg gct ggc      1838
Gly Ser Ser Tyr Thr Leu Ser Leu Ser Gly Ser Gly Tyr Ala Ala Gly
        435                 440                 445

acc aag ctg gtg gaa atg tac acc tgc acc gct gtt acc gtt gat tcg      1886
Thr Lys Leu Val Glu Met Tyr Thr Cys Thr Ala Val Thr Val Asp Ser
    450                 455                 460

aat ggc aac atc gca gtt tcc atg acc tct ggg ctg cct cga gtg ttt      1934
```

```
Asn Gly Asn Ile Ala Val Ser Met Thr Ser Gly Leu Pro Arg Val Phe
465                 470                 475                 480

atg ctg gcc tcc tct gca tgc tct ctt tgc agt tcc gcg tgc tcc gca       1982
Met Leu Ala Ser Ser Ala Cys Ser Leu Cys Ser Ser Ala Cys Ser Ala
                485                 490                 495

act gca acc acg ctc aag act acg acc gct acg gcg acc agc tgc acc       2030
Thr Ala Thr Thr Leu Lys Thr Thr Thr Ala Thr Ala Thr Ser Cys Thr
            500                 505                 510

caa gcc acc gct ctg cct gtt ctg ttt aaa gat aca gtg acc act tct       2078
Gln Ala Thr Ala Leu Pro Val Leu Phe Lys Asp Thr Val Thr Thr Ser
        515                 520                 525

tac ggt cag agc gtc tat ctc gcc ggc tcg atc agt cag ctc ggt aac       2126
Tyr Gly Gln Ser Val Tyr Leu Ala Gly Ser Ile Ser Gln Leu Gly Asn
    530                 535                 540

tgg aac gcc gct aat gct gtt gcg ttg tcc gct gat aag tac aca tca       2174
Trp Asn Ala Ala Asn Ala Val Ala Leu Ser Ala Asp Lys Tyr Thr Ser
545                 550                 555                 560

tcc aat ccc tta tgg tac gca act gtg acg ctg ccc gtg gga act tcg       2222
Ser Asn Pro Leu Trp Tyr Ala Thr Val Thr Leu Pro Val Gly Thr Ser
                565                 570                 575

ttc cag tac aag ttc atc aaa aag acg agc ggc tct ggc tcg gtc act       2270
Phe Gln Tyr Lys Phe Ile Lys Lys Thr Ser Gly Ser Gly Ser Val Thr
            580                 585                 590

tgg gag agc gac ccc aac cgg tcg tac aca gtc ccc acc gga tgc gtg       2318
Trp Glu Ser Asp Pro Asn Arg Ser Tyr Thr Val Pro Thr Gly Cys Val
        595                 600                 605

ggc tct acg gcc act gtc act gct acg tgg agg tag                       2354
Gly Ser Thr Ala Thr Val Thr Ala Thr Trp Arg
    610                 615
```

<210> 4
<211> 619
<212> PRT
<213> Penicillium oxalicum

<400> 4

```
Met Lys Phe Leu Gly Leu Ala Ala Leu Phe Leu Ala Gln Thr Val Ala
1               5                   10                  15

Gly Leu Thr Ala Ala Gln Trp Arg Ser Gln Ser Ile Tyr Phe Leu Met
            20                  25                  30

Thr Asp Arg Phe Gly Arg Thr Asp Lys Ser Val Thr Ala Pro Cys Asn
        35                  40                  45

Thr Asn Asp Arg Val Tyr Cys Gly Gly Thr Trp Gln Gly Ile Ile Asn
    50                  55                  60
```

```
Gln Leu Asp Tyr Ile Gln Gly Met Gly Phe Thr Ala Ile Trp Ile Thr
65                  70                  75                  80

Pro Val Thr Glu Gln Leu Pro Gln Asp Thr Gly Asp Gly Glu Ala Tyr
                85                  90                  95

His Gly Tyr Trp Gln Gln Glu Ile Tyr Asn Val Asn Asn Asn Tyr Gly
            100                 105                 110

Thr Ala Ala Asp Leu Lys Ala Leu Ser Gln Ala Leu His Ser Arg Gly
        115                 120                 125

Met Tyr Leu Met Val Asp Val Val Ala Asn His Met Gly Tyr Ala Gly
    130                 135                 140

Ala Gly Asn Thr Val Asp Tyr Ser Val Phe Lys Pro Phe Ser Ser Ser
145                 150                 155                 160

Ser Tyr Phe His Pro Tyr Cys Leu Ile Ser Asp Tyr Ser Asn Gln Thr
                165                 170                 175

Asn Val Glu Asp Cys Trp Leu Gly Asp Thr Thr Val Ser Leu Pro Asp
            180                 185                 190

Leu Asp Thr Thr Leu Ser Ser Val Gln Thr Ile Trp Tyr Asn Trp Val
        195                 200                 205

Ser Asp Leu Val Ser Asn Tyr Ser Ile Asp Gly Leu Arg Ile Asp Thr
    210                 215                 220

Val Lys His Val Gln Lys Ser Phe Trp Pro Gly Tyr Gln Ser Ala Ala
225                 230                 235                 240

Gly Val Tyr Cys Val Gly Glu Val Phe Ser Gly Asp Pro Ala Tyr Thr
                245                 250                 255

Cys Pro Tyr Gln Asn Tyr Leu Asp Gly Val Leu Asn Tyr Pro Ile Tyr
            260                 265                 270

Tyr Gln Leu Leu Gly Ala Phe Lys Ser Thr Ser Gly Ser Ile Ser Ser
        275                 280                 285

Leu Tyr Asn Met Ile Asn Ser Val Ala Ser Asp Cys Ala Asp Pro Thr
    290                 295                 300

Leu Leu Gly Asn Phe Ile Glu Asn His Asp Asn Pro Arg Phe Ala Ser
305                 310                 315                 320
```

```
Tyr Thr Ser Asp Tyr Ser Gln Ala Lys Asn Val Ile Ser Phe Ile Phe
                325                 330                 335

Leu Ser Asp Gly Ile Pro Ile Val Tyr Ala Gly Gln Glu Gln His Tyr
            340                 345                 350

Ser Gly Gly Asn Asp Pro Ala Asn Arg Glu Ala Thr Trp Leu Ser Gly
        355                 360                 365

Tyr Ser Lys Asn Ala Gln Leu Tyr Gln His Ile Ala Ser Thr Asn Lys
    370                 375                 380

Ile Arg Ser Leu Ala Ile Ser Lys Asp Ala Asn Tyr Ile Thr Ser Lys
385                 390                 395                 400

Asn Asn Ala Phe Tyr Thr Asp Ser Asn Thr Ile Ala Met Lys Lys Gly
                405                 410                 415

Ser Ser Gly Ser Gln Val Val Thr Val Leu Ser Asn Arg Gly Ser Ser
            420                 425                 430

Gly Ser Ser Tyr Thr Leu Ser Leu Ser Gly Ser Gly Tyr Ala Ala Gly
        435                 440                 445

Thr Lys Leu Val Glu Met Tyr Thr Cys Thr Ala Val Thr Val Asp Ser
    450                 455                 460

Asn Gly Asn Ile Ala Val Ser Met Thr Ser Gly Leu Pro Arg Val Phe
465                 470                 475                 480

Met Leu Ala Ser Ser Ala Cys Ser Leu Cys Ser Ser Ala Cys Ser Ala
                485                 490                 495

Thr Ala Thr Thr Leu Lys Thr Thr Thr Ala Thr Ala Thr Ser Cys Thr
            500                 505                 510

Gln Ala Thr Ala Leu Pro Val Leu Phe Lys Asp Thr Val Thr Thr Ser
        515                 520                 525

Tyr Gly Gln Ser Val Tyr Leu Ala Gly Ser Ile Ser Gln Leu Gly Asn
    530                 535                 540

Trp Asn Ala Ala Asn Ala Val Ala Leu Ser Ala Asp Lys Tyr Thr Ser
545                 550                 555                 560

Ser Asn Pro Leu Trp Tyr Ala Thr Val Thr Leu Pro Val Gly Thr Ser
                565                 570                 575
```

Phe Gln Tyr Lys Phe Ile Lys Lys Thr Ser Gly Ser Gly Ser Val Thr
580 585 590

Trp Glu Ser Asp Pro Asn Arg Ser Tyr Thr Val Pro Thr Gly Cys Val
595 600 605

Gly Ser Thr Ala Thr Val Thr Ala Thr Trp Arg
610 615

<210> 5
<211> 44
<212> DNA
<213> Artificial Sequence

<220>
<223> Synthetic construct

<400> 5
attattcgaa ggatccacca tgttcttcac atcctcggtc tgct 44

<210> 6
<211> 39
<212> DNA
<213> Artificial Sequence

<220>
<223> Synthetic construct

<400> 6
ggtgctgatg gaattcggaa taaccacaca atccgctgc 39

<210> 7
<211> 48
<212> DNA
<213> Artificial Sequence

<220>
<223> Synthetic construct

<400> 7
attattcgaa ggatccacca tgaaattcct tggactagct gctttgtt 48

<210> 8
<211> 39
<212> DNA
<213> Artificial Sequence

<220>
<223> Synthetic construct

<400> 8
ggtgctgatg gaattccctc cacgtagcag tgacagtgg 39

## Description

## Background of the Invention

### Field of the Invention

The present invention relates to polypeptides having alpha-amylase activity, catalytic domains, and carbohydrate binding domains, and polynucleotides encoding the polypeptides, catalytic domains, and carbohydrate binding domains. The invention also relates to nucleic acid constructs, vectors, and host cells comprising the polynucleotides as well as methods of producing and using the polypeptides, catalytic domains, and carbohydrate binding domains.

### Description of the Related Art

Alpha-amylases (alpha-1,4-glucan-4-glucanohydrolases, EC. 3.2.1.1) constitute a group of enzymes which catalyze hydrolysis of starch and other linear and branched 1,4-glucosidic oligo- and polysaccharides.
For a number of years alpha-amylase enzymes have been used for a variety of different purposes, the most important of which are starch liquefaction, textile desizing, textile washing, starch modification in the paper and pulp industry, and for brewing, ethanol production and baking.
The object of the present invention is to provide alpha-amylases for conversion of starch into maltodextrins, mono- and disaccharides and/or useful in processes involving starch liquefaction, textile washing, textile desizing, starch modification in the paper and pulp industry, and for brewing, ethanol production and baking.
A polypeptide from *Aspergillus fumigatus* having alpha-amylase activity is disclosed in WO 2003/012071 (GeneseqP:ABB80178). A polypeptide from *Aspergillus terreus* having alpha-amylase activity is disclosed in WO 2010/091221. A putative alpha-amylase from *Aspergillus flavus* is disclosed in UNIPROT:B8N2R4 B8N2R4_ASPFN. A putative alpha-amylase from *Neosartorya fischeri is* disclosed in UNIPROT:A1CYB1 A1CYB1_NEOFI.

## Summary of the Invention

The present invention relates to isolated polypeptides having alpha-amylase activity selected from the group consisting of:

(a) a polypeptide having at least 90%, at least 91 %, at least 92%, at least 93%, at least 94%, at least 95%, at least 96%, at least 97%, at least 98%, at least 99%, or 100% sequence identity to amino acids 21-619 of SEQ ID NO: 4; wherein % identity is determined using the Needle program of the EMBOSS package with a gap open penalty of 10, a gap extension penalty of 0.5, and the EBLOSUM62 by the calculation: (Identical Residues x 100)/ (Length of Alignment - Total Number of Gaps in Alignment).

The present invention also relates to isolated polypeptides comprising a catalytic domain selected from the group consisting of:

(a) a catalytic domain having at least 95% sequence identity to amino acids 21 to 493 of SEQ ID NO: 4.

The present invention also relates to isolated polynucleotides encoding the polypeptides of the present invention;and methods of producing the polypeptides.
The present invention also relates to use of the present alpha-amylase for starch conversion, starch modification in the paper and pulp industry, starch liquefaction, textile washing, textile desizing, brewing, ethanol production and/or baking.
The present invention also relates to a composition comprising the polypeptide of the present invention and an enzyme selected from the group consisting of: a fungal alpha-amylase (EC 3.2.1.1), a beta-amylase (E.C. 3.2.1.2), a glucoamylase (E.C.3.2.1.3), a pullulanases (E.C. 3.2.1.41), a phytase (E.C.3.1.2.28) and a protease (E.C. 3.4.).
The present invention also relates to a whole broth formulation or cell culture composition comprising the polypeptide of of the invention.

## Brief Description of the Figures

Figure 1 shows a construct of P243SV and pLIZG8HQ.

Figure 2 shows a construct of P23WU1 and pLIZG8HQ.

**Definitions**

**alpha-amylase:** The term "alpha-amylase" means an alpha-amylase activity (E.C. 3.2.1.1) that catalyzes the endohydrolysis of (1→4)-alpha-D-glucosidic linkages in polysaccharides containing three or more (1→4)-alpha-linked D-glucose units. The term "alpha-amylase activity" corresponds to the enzymes grouped in E.C. 3.2.1.1. For purposes of the present invention, alpha-amylase activity is determined according to the procedure described in the Examples. In one aspect, the polypeptides of the present invention have at least 20%, *e.g.,* at least 40%, at least 50%, at least 60%, at least 70%, at least 80%, at least 90%, at least 95%, or at least 100% of the alpha-amylase activity of the mature polypeptide of SEQ ID NO: 2, or the mature polypeptide of SEQ ID NO: 4.

**Glucoamylase activity:** The term "glucoamylase (1,4-alpha-D-glucan glucohydrolase, EC 3.2.1.3) activity" is defined herein as an enzyme activity, which catalyzes the release of D-glucose from the non-reducing ends of starch or related oligo- and poly-saccharide molecules. Glucoamylase activity may be measured in AGU units.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Carbohydrate binding domain:** The term "carbohydrate binding domain" or "CBD" is defined herein as an amino acid sequence comprising a CBD of family 20, also known as a starch binding domain (SBD). In SEQ ID NO: 4, amino acids 511 to 619 are the CBD.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (*i.e.*, from the same gene) or foreign (*i.e.*, from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Fragment:** The term "fragment" means a polypeptide or a catalytic or carbohydrate binding domain having one or more (*e.g.*, several) amino acids absent from the amino and/or carboxyl terminus of a mature polypeptide or domain; wherein the fragment has alpha-amylase or carbohydrate binding activity. In one aspect, a fragment contains at least 508 amino acid residues, preferably at least 538 amino acid residues, more preferably 568 amino acid residues of SEQ ID NO: 4.

In one specific embodiment a fragment comprises amino acids 21 to 493 of SEQ ID NO: 4. In one specific embodiment a fragment comprises amino acids 511 to 619 of SEQ ID NO: 4.

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.*, recombinant production in a host cell; multiple copies of a gene encoding the substance; and use of a stronger promoter than the promoter naturally associated with the gene encoding the substance).

**Mature polypeptide:** The term "mature polypeptide" means a polypeptide in its final form following translation and any post-translational modifications, such as N-terminal processing, C-terminal truncation, glycosylation, phosphorylation, *etc.* In one aspect, the mature polypeptide is amino acids 21 to 619 of SEQ ID NO: 4, based on the programs (*e.g.,* SignalP (Nielsen et al., 1997, Protein Engineering 10: 1-6)) that predicts amino acids 1 to 20 of SEQ ID NO: 4 are signal peptides. It is known in the art that a host cell may produce a mixture of two of more different mature polypeptides (*i.e*., with a different C-terminal and/or N-terminal amino acid) expressed by the same polynucleotide.

**Mature polypeptide coding sequence:** The term "mature polypeptide coding sequence" means a polynucleotide that encodes a mature polypeptide having alpha-amylase activity. In one aspect, the mature polypeptide coding sequence is nucleotides 61 to 2351 of SEQ ID NO: 3, or nucleotides 76 to 1584 of SEQ ID NO: 1, or the cDNA sequence thereof, based on the program *e.g.,* SignalP (Nielsen *et al.,* 1997, *supra)* that predicts nucleotides 1 to 60 of SEQ ID NO: 3 encode a signal peptide.

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and either 35% formamide, following standard Southern blotting procedures for 12 to 24 hours. The carrier material is finally washed three times each for 15 minutes using 2X SSC, 0.2% SDS at 60°C.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice *et al.,* 2000, *supra),* preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** The term "subsequence" means a polynucleotide having one or more (*e.g.*, several) nucleotides absent from the 5' and/or 3' end of a mature polypeptide coding sequence; wherein the subsequence encodes a fragment having alpha-amylase activity. In one aspect, a subsequence contains at least 1524 nucleotides, preferably at least 1614 nucleotides, more preferably at least 1704 nucleotides of SEQ ID NO: 3.

**Variant:** The term "variant" means a polypeptide having alpha-amylase activity comprising an alteration, *i.e*., a substitution, insertion, and/or deletion, at one or more (*e.g.,* several) positions. A substitution means replacement of the amino acid occupying a position with a different amino acid; a deletion means removal of the amino acid occupying a position; and an insertion means adding an amino acid adjacent to and immediately following the amino acid occupying a position.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

**Very low stringency conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

**Detailed Description of the Invention**

**Polypeptides Having alpha-amylase Activity**

In an embodiment, the present invention relates to isolated polypeptides having a sequence identity to the mature polypeptide of SEQ ID NO: 4 of at least 90%, at least 91%, at least 92%, at least 93%, at least 94%, at least 95%, at least 96%, at least 97%, at least 98%, at least 99%, or 100%, which have alpha-amylase activity.

In one aspect, the polypeptides differ by no more than 10 amino acids, *e.g.,* 1, 2, 3, 4, 5, 6, 7, 8, 9 or 10, from the mature polypeptide of SEQ ID NO: 4.

A polypeptide of the present invention preferably comprises or consists of the amino acid sequence of SEQ ID NO: 4. In another aspect, the polypeptide comprises or consists of the mature polypeptide of SEQ ID NO: 4. In another aspect, the polypeptide comprises or consists of amino acids 21 to 619 of SEQ ID NO: 4.

In another embodiment, the present disclosure relates to an isolated polypeptide having alpha-amylase activity encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 3, (ii) the cDNA sequence thereof, or (iii) the full-length complement of (i) or (ii) (Sambrook et al., 1989, Molecular Cloning, A Laboratory Manual, 2d edition, Cold Spring Harbor, New York).

The polynucleotide of SEQ ID NO: 3 or a subsequence thereof, as well as the polypeptide of SEQ ID NO: 4, or a fragment thereof, may be used to design nucleic acid probes to identify and clone DNA encoding polypeptides having alpha-amylase activity from strains of different genera or species according to methods well known in the art. In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, *e.g.,* at least 25, at least 35, or at least 70 nucleotides in length. Preferably, the nucleic acid probe is at least 100 nucleotides in length, *e.g.,* at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from such other strains may be screened for DNA that hybridizes with the probes described above and encodes a polypeptide having alpha-amylase activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that hybridizes with SEQ ID NO: 3 or the mature polypeptide coding sequence thereof, or a subsequence thereof, the carrier material is used in a Southern blot.

For purposes of the present disclosure, hybridization indicates that the polynucleotide hybridizes to a labeled nucleic acid probe corresponding to (i) SEQ ID NO: 3; (ii) the mature polypeptide coding sequence of SEQ ID NO: 3; (iii) the cDNA sequence thereof; (iv) the full-length complement thereof; or (v) a subsequence thereof; under very low to very high stringency conditions. Molecules to which the nucleic acid probe hybridizes under these conditions can be detected using, for example, X-ray film or any other detection means known in the art.

In one aspect, the nucleic acid probe is nucleotides 61 to 2351 of SEQ ID NO: 3. In another aspect of the diclosure, the nucleic acid probe is a polynucleotide that encodes the polypeptide of SEQ ID NO: 4; the mature polypeptide thereof; or a fragment thereof. In another aspect, the nucleic acid probe is SEQ ID NO: 3, or the cDNA sequence thereof.

In another embodiment, the present disclosure relates to an isolated polypeptide having alpha-amylase activity encoded by a polynucleotide having a sequence identity to the mature polypeptide coding sequence of SEQ ID NO: 3 or the cDNA sequence thereof of at least 80%, *e.g.,* at least 85%, at least 90%, at least 91%, at least 92%, at least 93%, at least 94%, at least 95%, at least 96%, at least 97%, at least 98%, at least 99%, or 100%. In another embodiment, the present diclodure relates to variants of the mature polypeptide of SEQ ID NO: 4 comprising a substitution, deletion, and/or insertion at one or more (*e.g.,* several) positions. In an embodiment, the number of amino acid substitutions, deletions and/or insertions introduced into the mature polypeptide of SEQ ID NO: 4 is at most 10, *e.g.,* 1, 2, 3, 4, 5, 6, 7, 8, 9 or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for alpha-amylase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et a/., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

In one aspect, the polypeptides having alpha-amylase activity of the present invention show very good thermostability. The polypeptides having alpha-amylase activity of the present inventions are stable at 10 °C- 100 °C, preferably 20 °C- 80 °C, more preferably 30 °C- 70 °C.

**Sources of Polypeptides Having alpha-amylase Activity**

A polypeptide having alpha-amylase activity of the present invention may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

The polypeptide may be a fungal polypeptide. In another aspect, the polypeptide is a *Penicillium* polypeptide, e.g., a polypeptide obtained from *Penicillium oxalicum, Penicillium funiculosum,* or *Penicillium purpurogenum.*

It will be understood that for the aforementioned species, the invention encompasses both the perfect and imperfect states, and other taxonomic equivalents, *e.g.,* anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature (*e.g.,* soil, composts, water, etc.) or DNA samples obtained directly from natural materials (*e.g.,* soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.,* Sambrook *et al.,* 1989, *supra*).

**Catalytic Domains**

In one embodiment, the present invention also relates to catalytic domains having a sequence identity to amino acids 21 to 493 of SEQ ID NO: 4 of at least 95%, at least 96%, at least 97%, at least 98%, at least 99%, or 100%. In one aspect, the catalytic domains comprise amino acid sequences that differ by up to 10 amino acids, e.g., 1, 2, 3, 4, 5, 6, 7, 8, 9, or 10, from amino acids 21 to 493 of SEQ ID NO: 4.

The catalytic domain preferably comprises or consists of amino acids 21 to 493 of SEQ ID NO: 4.

In another embodiment, the present disclosure also relates to catalytic domains encoded by polynucleotides that hybridize under very low stringency conditions, low stringency conditions, medium stringency conditions, medium-high stringency conditions, high stringency conditions, or very high stringency conditions (as defined above) with (i) nucleotides 61 to 1973 of SEQ ID NO: 3, (ii) the cDNA sequence thereof, or (iii) the full-length complement of (i) or (ii) (Sambrook *et al.,* 1989, *supra).*

In another embodiment, the present disclosure also relates to catalytic domains encoded by polynucleotides having a sequence identity to nucleotides 61 to 1973 of SEQ ID NO: 3 of at least 80%, *e.g.,* at least 85%, at least 90%, at least 95%, at least 96%, at least 97%, at least 98%, at least 99%, or 100%, or the cDNA sequence thereof. The polynucleotide encoding the catalytic domain preferably comprises or consists of nucleotides 61 to 1973 of SEQ ID NO: 3.

**Binding Domains**

In one embodiment, the present disclosure also relates to carbohydrate binding domains having a sequence identity to amino acids 511 to 619 of SEQ ID NO: 4 of at least 80%, *e.g.,* at least 85%, at least 90%, at least 91 %, at least 92%, at least 93%, at least 94%, at least 95%, at least 96%, at least 97%, at least 98%, at least 99%, or 100%. In one aspect, the carbohydrate binding domains comprise amino acid sequences that differ by up to 10 amino acids, *e.g.,* 1, 2, 3, 4, 5, 6, 7, 8, 9, or 10, from amino acids 511 to 619 of SEQ ID NO: 4.

The carbohydrate binding domain preferably comprises or consists of amino acids 511 to 619 of SEQ ID NO: 4 or an allelic variant thereof; or is a fragment thereof having carbohydrate binding activity.

In another embodiment, the present disclosure also relates to carbohydrate binding domains encoded by polynucleotides that hybridize under very low stringency conditions, low stringency conditions, medium stringency conditions, medium-high stringency conditions, high stringency conditions, or very high stringency conditions (as defined above) with (i) the nucleotides 2025 to 2351 of SEQ ID NO: 3 (ii) the cDNA sequence thereof, or (iii) the full-length complement of (i) or (ii) (Sambrook *et al.,* 1989, *supra).*

In another embodiment, the present disclosure also relates to carbohydrate binding domains encoded by polynucleotides having a sequence identity to nucleotides 2025 to 2351 of SEQ ID NO: 3 of at least 80%, *e.g.,* at least 85%, at least 90%, at least 91%, at least 92%, at least 93%, *e.g.,* at least 94%, at least 95%, at least 96%, at least 97%, at least 98%, at least 99%, or 100%.

The polynucleotide encoding the carbohydrate binding domain preferably comprises or consists of nucleotides 2025 to 2351 of SEQ ID NO: 3.

In another embodiment, the present disclosure also relates to carbohydrate binding domain variants of amino acids 511 to 619 of SEQ ID NO: 4 comprising a substitution, deletion, and/or insertion at one or more (*e.g.,* several) positions. In one aspect, the number of amino acid substitutions, deletions and/or insertions introduced into the sequence of amino acids 511 to 619 of SEQ ID NO: 4 is up to 10, *e.g.,* 1, 2, 3, 4, 5, 6, 8, 9, or 10.

A catalytic domain operably linked to the carbohydrate binding domain may be from an amylase, preferably an alpha-amylase, more preferably an acid alpha-amylase. The polynucleotide encoding the catalytic domain may be obtained from any prokaryotic, eukaryotic, or other source.

**Polynucleotides**

The present invention also relates to isolated polynucleotides encoding a polypeptide, a catalytic domain, or carbohydrate binding domain of the present invention, as described herein.

The techniques used to isolate or clone a polynucleotide are known in the art and include isolation from genomic DNA or cDNA, or a combination thereof. The cloning of the polynucleotides from genomic DNA can be effected, *e.g.,* by using the well known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of *Penicillium* polypeptide, or a related organism and thus, for example, may be an allelic or species variant of the polypeptide encoding region of the polynucleotide.

Modification of a polynucleotide encoding a polypeptide of the present invention may be necessary for synthesizing polypeptides substantially similar to the polypeptide. The term "substantially similar" to the polypeptide refers to non-naturally occurring forms of the polypeptide. These polypeptides may differ in some engineered way from the polypeptide isolated from its native source, *e.g.,* variants that differ in specific activity, thermostability, pH optimum, or the like. The variants may be constructed on the basis of the polynucleotide presented as the mature polypeptide coding sequence of SEQ ID NO: 3, or the cDNA sequence thereof, *e.g.,* a subsequence thereof, and/or by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, *e.g.,* Ford et al., 1991, Protein Expression and Purification 2: 95-107.

**Nucleic Acid Constructs**

The present disclosure also relates to nucleic acid constructs comprising a polynucleotide of the present invention operably linked to one or more control sequences that direct the expression of the coding sequence in a suitable host cell under conditions compatible with the control sequences.

A polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase *I, Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (*aprH*), *Bacillus licheniformis* alpha-amylase (*amyL*), and *Escherichia coli* ribosomal RNA (*runB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

**Expression Vectors**

The present disclosure also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.,* a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.*, a vector that exists as an extrachromosomal entity, the replication of which is independent of chromosomal replication, *e.g.,* a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is a *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMß1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook *et al.,* 1989, *supra).*

## Host Cells

The present invention also relates to recombinant host cells, comprising a polynucleotide of the present invention operably linked to one or more control sequences that direct the production of a polypeptide of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, *e.g.,* a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi subsp. Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth *et al., In, Ainsworth and Bisby's Dictionary of The Fungi,* 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharo-*

*myces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

## Methods of Production

The present invention also relates to methods of producing a polypeptide of the present invention, comprising (a) cultivating a cell, which in its wild-type form produces the polypeptide, under conditions conducive for production of the polypeptide; and optionally (b) recovering the polypeptide. In a preferred aspect, the cell is a *Penicillium* cell. In a more preferred aspect, the cell is *Penicillium oxalicum* cell.

The present invention also relates to methods of producing a polypeptide of the present invention, comprising (a) cultivating a recombinant host cell of the present invention under conditions conducive for production of the polypeptide; and optionally (b) recovering the polypeptide.

The host cells are cultivated in a nutrient medium suitable for production of the polypeptide using methods known in the art. For example, the cells may be cultivated by shake flask cultivation, or small-scale or large-scale fermentation (including continuous, batch, fed-batch, or solid state fermentations) in laboratory or industrial fermentors in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, a whole fermentation broth comprising the polypeptide is recovered.

The polypeptide may be purified by a variety of procedures known in the art including, but not limited to, chromatography (*e.g.,* ion exchange, affinity, hydrophobic, chromatofocusing, and size exclusion), electrophoretic procedures (*e.g.,* preparative isoelectric focusing), differential solubility (*e.g.,* ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the polypeptide is not recovered, but rather a host cell of the present invention expressing the polypeptide is used as a source of the polypeptide.

**Fermentation Broth Formulations or Cell Compositions**

The present invention also relates to a fermentation broth formulation or a cell composition comprising a polypeptide of the present invention. The fermentation broth product further comprises additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.,* expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.,* filamentous fungal cells) are removed, *e.g.,* by centrifugation. In some embodiments, the fermentation broth contains spent cell culture medium, extracellular enzymes, and viable and/or nonviable microbial cells.

In an embodiment, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific embodiment, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one embodiment, the killed cells and/or cell debris are removed from a cell-killed whole broth to provide a composition that is free of these components.

The fermentation broth formulations or cell compostions may further comprise a preservative and/or antimicrobial (*e.g.,* bacteriostatic) agent, including, but not limited to, sorbitol, sodium chloride, potassium sorbate, and others known in the art.

The fermentation broth formulations or cell compositions may further comprise multiple enzymatic activities, such as one or more (*e.g.,* several) enzymes selected from the group comprising of; a fungal alpha-amylase (EC 3.2.1.1), a beta-amylase (E.C. 3.2.1.2), a glucoamylase (E.C.3.2.1.3), a pullulanases (E.C. 3.2.1.41 a phytase (E.C.3.1.2.28) and a protease (E.C. 3.4.). The glucoamylase may preferably be derived from a strain of *Aspergillus* sp., such as *Aspergillus niger,* or from a strain of *Talaromyces* sp. and in particular derived from *Talaromyces leycettanus* such as the glucoamylase disclosed in U.S. patent no. Re. 32,153, *Talaromyces duponti* and/or *Talaromyces thermopiles* such as the glucoamylases disclosed in U.S. Patent No. 4,587,215 and more preferably derived from *Talaromyces emersonii.* Most preferably the glucoamylase is derived from *Talaromyces emersonii* strain CBS 793.97 and/or having the sequence disclosed as SEQ ID NO: 7 in WO 99/28448. Further preferred is a glucoamylase which has an amino acid sequence having at least 50%, at least 60%, at least 70%, at least 80%, at least 90%, at least 95%, at least 96%, at least 97%, at least 98%, or even at least 99% identity to the aforementioned amino acid sequence.

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.,* filamentous fungal cells) are grown to saturation, incubated under carbon-limiting conditions to allow protein synthesis (*e.g.,* expression of cellulase and/or glucosidase enzyme(s)). In some embodiments, the cell-killed whole broth or composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

Examples are given below of preferred uses of the compositions of the present invention. The dosage of the composition and other conditions under which the composition is used may be determined on the basis of methods known in the art.

**Enzyme Compositions**

The present invention also relates to compositions comprising a polypeptide of the present invention. Preferably, the compositions are enriched in such a polypeptide. The term "enriched" indicates that the cellulolytic enhancing activity of the composition has been increased, *e.g.,* with an enrichment factor of at least 1.1.

The compositions may comprise a polypeptide of the present invention as the major enzymatic component, *e.g.,* a mono-component composition. Alternatively, the compositions may comprise multiple enzymatic activities, such as one or more (*e.g.,* several) enzymes selected from the group comprising of; a fungal alpha-amylase (EC 3.2.1.1), a beta-amylase (E.C. 3.2.1.2), a glucoamylase (E.C.3.2.1.3), a pullulanases (E.C. 3.2.1.41), a phytase (E.C.3.1.2.28) and a protease (E.C. 3.4.). The glucoamylase may preferably be derived from a strain of *Aspergillus* sp., such as *Aspergillus niger,* or from a strain of *Talaromyces* sp. and in particular derived from *Talaromyces leycettanus* such as the glucoamylase disclosed in U.S. patent no. Re. 32,153, *Talaromyces duponti* and/or *Talaromyces thermopiles* such as the glucoamylases disclosed in U.S. Patent No. 4,587,215 and more preferably derived from *Talaromyces emersonii.* Most preferably the glucoamylase is derived from *Talaromyces emersonii* strain CBS 793.97 and/or having the sequence disclosed as SEQ ID NO: 7 in WO 99/28448. Further preferred is a glucoamylase which has an amino acid sequence having at least 50%, at least 60%, at least 70%, at least 80%, at least 90%, at least 95%, at least 96%, at least 97%, at least 98%, or even at least 99% identity to the aforementioned amino acid sequence. A commercial *Talaromyces* glucoamylase preparation is supplied by Novozymes A/S as SPIRIZYME FUEL.

Also preferred for a composition comprising the polypeptide of the present invention and a glucoamylase are polypeptides having glucoamylase activity which are derived from a strain of the genus *Trametes,* preferably *Trametes cingulata.* Further preferred is polypeptide having glucoamylase activity and havering at least 50%, at least 60%, at least 70%, at least 80%, at least 90% or even at least 95% identity with amino acids for mature polypeptide of SEQ ID NO: 2 in WO 2006/069289.

Also preferred for a composition comprising the polypeptide of the present invention and a glucoamylase are polypeptides having glucoamylase activity which are derived from a strain of the genus *Pachykytospora,* preferably *Pachykytospora papyracea* or the *E. coli* strain deposited at DSMZ and given the no. DSM 17105. Further preferred are polypeptides having glucoamylase activity and having at least 50%, at least 60%, at least 70%, at least 80%, at least 90% or even at least 95% identity with amino acids for mature polypeptide of SEQ ID NO: 5 in WO 2006/069289.

The composition described above may preferably comprise acid alpha-amylase present in an amount of 0.01 to 10 AFAU/g DS, preferably 0.1 to 5 AFAU/g DS, more preferably 0.15 to 3 AFAU/g DS, and most preferably 0.3 to 2 AFAU/g DS. The dosage of the polypeptide composition of the invention and other conditions under which the composition is used may be determined on the basis of methods known in the art.

The polypeptide compositions may be prepared in accordance with methods known in the art and may be in the form of a liquid or a dry composition. For instance, the polypeptide composition may be in the form of granulate or a microgranulate. The polypeptide to be included in the composition may be stabilized in accordance with methods known in the art.

**Uses**

The present invention is also directed to methods for using the polypeptides having alpha-amylase activity, or compositions thereof.

The polypeptide or the composition of the present invention may be used in starch conversion, starch to sugar conversion and ethanol production etc, e.g., in liquefying and/or saccharifying a gelatinized starch or a granular starch, as well as a partly gelatinized starch. A partly gelatinized starch is a starch which to some extent is gelatinized, *i.e.,* wherein part of the starch has irreversibly swelled and gelatinized and part of the starch is still present in a granular state.It can be used in a process for liquefying starch, wherein a gelatinized or granular starch substrate is treated in aqueous medium with the enzyme. The polypeptide or the composition of the present invention may also be used in a process for saccharification of a liquefied starch substrate. A preferred use is in a fermentation process wherein a starch substrate is liquefied and/or saccharified in the presence of the polypeptide or the composition of the present invention to produce glucose and/or maltose suitable for conversion into a fermentation product by a fermenting organism, preferably a yeast. Such fermentation processes include a process for producing ethanol for fuel or drinking ethanol (portable alcohol), a process for producing a beverage, a process for producing desired organic compounds, such as citric acid, itaconic acid, lactic acid, gluconic acid, sodium gluconate, calcium gluconate, potassium gluconate, glucono delta lactone, or sodium erythorbate; ketones; amino acids, such as glutamic acid (sodium monoglutaminate), but also more complex compounds such as antibiotics, such as penicillin, tetracyclin; enzymes; vitamins, such as riboflavin, B12, beta-carotene; hormones, which are difficult to produce synthetically.

Furthermore, due to the superior hydrolysis activity of the polypeptide of the present invention, the amount of glucoamylase during the saccharification step can be reduced. The glucoamylase may preferably be derived from a strain within *Aspergillus* sp., *Talaromyces* sp., *Pachykytospora* sp. or *Trametes* sp., more preferably from *Aspergillus niger, Talaromyces emersonii, Trametes cingulata* or *Pachykytospora papyracea.*

In a preferred embodiment, the polypeptide of the present invention is used in a process comprising fermentation to produce a fermentation product, *e.g.,* ethanol, from a gelatinized starch. Such a process for producing ethanol from gelatinized starch by fermentation comprises:

(i) liquefying the gelatinized starch with a polypeptide with alpha-amylase activity of the present invention; (ii) saccharifying the liquefied mash obtained; (iii) fermenting the material obtained in step (ii) in the presence of a fermenting organism. Optionally the process further comprises recovery of the ethanol. The saccharification and fermentation may be carried out as a simultaneous saccharification and fermentation process (SSF process).

In another preferred embodiment, the polypeptide of the present invention is used in a process comprising fermentation to produce a fermentation product, *e.g.,* ethanol, from an ungelatinized ("raw") starch. Such a process for producing ethanol from ungelatinized starch-containing material by fermentation comprises: (i) contacting the ungelatinized starch with a polypeptide with alpha-amylase activity of the present invention to degrade the ungelatinized starch; (ii) saccharifying the mash obtained; (iii) fermenting the material obtained in step (ii) in the presence of a fermenting organism. Optionally the process further comprises recovery of the ethanol. The saccharification and fermentation may be carried out as a simultaneous saccharification and fermentation process (SSF process).

In further embodiments, the polypeptide of the present invention may also be useful in textile, fabric or garment desizing or washing, in baking, detergent and pulp and paper production.

In other aspect, the present disclosure provides a method of using the alpha-amylase of the present invention for producing glucose or maltose or the like from starch.

The alpha-amylases of the invention may also be used in brewing processes. Further, the alpha-amylase of the invention may be used for maltose production. High maltose syrup is typically produced as follows. To produce "High Maltose Syrup" (containing 50-55% maltose), starch is liquefied to DE 10-20. The pH and temperature of the liquefied starch is adjusted to 65°C and to a pH around 5.0, respectively, and is subjected to maltogenic alpha-amylase activity (*e.g., Bacillus stearothermophilus* amylase, such as Maltogenase™ 4000 L, 0.4 l/t DS (Novozymes)), pullulanase activity *(e.g., Bacillus pullulanase,* such as Promozyme™ 600 L, 0.3 l/t DS (Novozymes)) and alpha-amylase activity (*e.g.,* BAN 240 L or Termamyl™ 120 L, type LS, 0.4 kg/t DS (Novozymes)) for 24-41 hours. The specific process time depends on the desired saccharide spectrum to be achieved. By increasing the dosage of the maltogenic alpha-amylase and pullulanase the maltose content can be increased.

Alternatively, "High Maltose Syrup" may be produced by first liquefying starch to DE 10-20 and then adjusting the pH and temperature to 55°C or higher and a pH around 5.5 or lower, and then subjecting the liquefied starch to a fungal alpha-amylase activity (*e.g., Bacillus stearothermophilus* amylase, such as Fungamyl™ 800L (Novozymes)) for 22-44 hours. The dosage of fungal Fungamyl™ 800L depends on the saccharification time foreseen, e.g., 200 g/t DS for 44 hours and 400 g/t DS for 22 hours. The alpha-amylases of the invention may substitute the Fungamyl™ 800L in the above process, and then the temperature can be even higher, and the pH even lower, resulting in a faster conversion rate, and thus a better overall economy.

To produce "High Maltose Syrup" starch with maltose content of 55-65% starch is liquefied to DE 10-20. The temperature and pH of the liquefied starch is adjusted to 60°C or higher, and to a pH around 6 or lower, and is subjected to maltogenic alpha-amylase activity (*e.g.,* Maltogenase™ 4000 L, 0.25-1.0 l/t DS (Novozymes)), and fungal alpha-amylase activity (*e.g.*, *Aspergillus* amylase, *such as* Fungamyl™ 800 L, 0.4-1.0 kg/t DS (Novo Nordisk) for 24-48 hours; or the alpha-amylase of the present invention for a shorter time.

The present invention is further described by the following examples.

**Examples**

**Materials**

Chemicals used as buffers and substrates were commercial products of at least reagent grade.

**Strains**

The fungal strain NN051380 was isolated from a soil sample collected from China with PDA medium at 25 °C using the dilution plate method. It was then purified by transferring a single conidium onto a YG agar plate. The strain NN051380 was identified as *Penicillium oxalicum* based on both morphological characteristics and ITS rDNA sequence.

**Media**

PDA medium was composed of 39 grams of potato dextrose agar and deionized water to 1 liter.
YG agar plates were composed of 5.0 g of yeast extract, 10.0 g of glucose, 20.0 g of agar, and deionized water to 1 liter.
YPG medium contained 0.4% of yeast extract, 0.1% of KH2PO4, 0.05% of $MgSO_4.7H_2O$, 1.5% glucose in deionized water.
YPM medium contained 1% yeast extract, 2% of peptone, and 2% of maltose in deionized water.
MD medium was composed of 1.34% YNB, $4X10^{-5}$% biotin and 2% dextrose. For plates, 7.5 g agar was added to 200ml of water autoclave, cooled to 60°C and then 25 ml of 10X YNB, 25ml of 10 X D-glucose and 400 $\mu$l of 500X biotin were added.
BMSY was composed of 1% yeast extract, 2% peptone (Bacto), 100mM potassium phosphate buffer, pH 6.0, 1.34% YNB, $4X10^{-5}$% biotin and 1.82% Sorbitol.
10 g of yeast extract, 20 g peptone (Bacto) and 18.2g Sorbitol were dissolved in 800 ml water and autoclaved for 20 minutes on liquid cycle. When the autoclaved medium was cooled to room temperature, 100 ml of 1 M potassium phosphate buffer (pH 6.0) and 100 ml of 10X YNB and 2 ml of 500X biotin were added.

**Determination of alpha-amylase activity**

The activity of any acid alpha-amylase may be measured in AFAU (Acid Fungal Alpha-amylase Units), which are determined relative to an enzyme standard. 1 AFAU is defined as the amount of enzyme which degrades 5.260 mg starch dry matter per hour under the below mentioned standard conditions.
Acid alpha-amylase, *i.e.,* acid stable alpha-amylase, an endo-alpha-amylase (1,4-alpha-D-glucan-glucanohydrolase, E.C. 3.2.1.1) hydrolyzes alpha-1,4-glucosidic bonds in the inner regions of the starch molecule to form dextrins and oligosaccharides with different chain lengths. The intensity of color formed with iodine is directly proportional to the concentration of starch. Amylase activity is determined using reverse colorimetry as a reduction in the concentration of starch under the specified analytical conditions.
Reaction condition: 10 microliters standard or enzyme sample, 70 microliters $H_2O$, and 80 microliters starch working solution (The final concentration was starch 0.35 g/L, Acetate buffer 50 mM pH 5.0, NaCl 0.1 M, $CaCl_2$ 3 mM) mixed and react for 2 mintues with shaking at 37°C. Add 40 microliters Iodine working solution (the final iodine concentration was 0.04 g/L) and react at 37°C for 1 minute. Reading $OD_{590}$ (Before reading, shaking 10 seconds).
FUNGAMYL™ (available from Novozymes A/S) is used as standard.

Example 1: *Penicillium oxalicum* genomic DNA extraction

*Penicillium oxalicum* strain NN051380 was inoculated onto a PDA plate and incubated for 3 days at 25°C in the darkness. Several mycelia-PDA plugs were inoculated into 500 ml shake flasks containing 100 ml of YPG medium. The flasks were incubated for 3 days at 25°C with shaking at 160 rpm. The mycelia were collected by filtration through MIRACLOTH® (Calbiochem, La Jolla, CA, USA) and frozen in liquid nitrogen. Frozen mycelia were ground, by a mortar and a pestle, to a fine powder, and genomic DNA was isolated using Large-Scale Column Fungal DNAout (BAOMAN BIOTECHNOLOGY, Shanghai, China) following the manufacturer's instruction.

Example 2: Genome sequencing, assembly and annotation

The extracted genomic DNA samples were delivered to Beijing Genome Institute (BGI, Shenzhen, China) for genome sequencing using ILLUMINA® GA2 System (Illumina, Inc., San Diego, CA, USA). The raw reads were assembled at BGI using program SOAPdenovo (Li et al. De novo assembly of human genomes with massively parallel short read sequencing. Genome Res (2010) vol. 20 (2) pp. 265-72). The assembled sequences were analyzed using standard bioinformatics methods for gene finding and functional prediction. Briefly, geneID (Parra et al., 2000, Genome Research 10(4):511-515) was used for gene prediction. Blastall version 2.2.10 (Altschul SF, Gish W, Miller W, Myers EW, Lipman DJ (October 1990). "Basic local alignment search tool". J Mol Biol 215 (3): 403-410, http://blast.ncbi.nlm.nih.gov/Blast.cgi) and HMMER version 2.1.1 (http://hmmer.janelia.org/) were used to predict function based on structural homology. The family GH13 enzyme polypeptides were identified directly by analysis of the Blast results. Agene program (Munch K, Krogh A. Automatic generation of gene finders for eukaryotic species. BMC Bioinformatics. 2006; 7: 263) and SignalP program (Nielsen et al., 1997, Protein Engineering 10: 1-6) were used to identify starting codons. SignalP program was further used to estimate length of signal peptide. Pepstats program (Rice P, Longden I, Bleasby A: EMBOSS: The European Molecular Biology Open Software Suite. Trends Genet. 2000, 16(6):276-277) was used to estimate isoelectric point of proteins, and molecular weight.

Example 3: Preparation of *Penicillium oxalicum* strain total RNA and cDNA

Total RNA was prepared from the powdered mycelia by using RNeasy plant mini kit (QIAGEN, Cat. No.74904). The cDNA was synthesized by following the instruction of 3' Rapid Amplifiction of cDNA End System (Invitrogen Corp., Carlsbad, CA, USA).

Example 4: Cloning of the *Penicillium oxalicum* alpha-amylase genes from cDNA

Two genes, S1 (P243SV, corresponding to SEQ ID NO: 1) and S2 (P23WU1, corresponding to SEQ ID NO: 3), identified as GH13 alpha-amylase were selected for expression cloning.

Based on the DNA information obtained from genome sequencing, oligonucleotide primers, shown below in table 1, were designed to amplify all from cDNA of *Penicillium oxalicum.* Primers fabricated by Invitrogen (Invitrogen, Beijing, China).

Table 1: Primers

| Primers | Sequence (5'-3') |
|---|---|
| S1_forward (SEQ ID NO: 5) | ATTATTCGAAGGATCCACC atgttcttcacatcctcggtctgct |
| S1_reverse (SEQ ID NO: 6) | GGTGCTGATGGAATTC ggaataaccacacaatccgctgc |
| S2_forward (SEQ ID NO: 7) | ATTATTCGAAGGATCCACC atgaaattccttggactagctgctttgtt |
| S2_reverse (SEQ ID NO: 8) | GGTGCTGATGGAATTC cctccacgtagcagtgacagtgg |

Lowercase characters represent the coding regions of the genes, while capitalized parts were homologous to the insertion sites of pLIZG8HQ vector. The expression vector pLIZG8HQ contained the *a*-factor secretion signal derived from *S. cerevisiae,* the 5'AOX1 promoter derived from *Pichia pastoris* and the 3'AOX1 alcohol oxidase1 terminator elements. Furthermore pLIZG8HQ had pBR322 derived sequences for selection and propagation in *E. coli,* and a *His*4 gene, which encoded an histidinol dehydrogenase derived from *Pichia pastoris* for selection of a transformant of a *His* mutant *Pichia* strain.

For each gene, 20 picomoles of primer pair (each of the forward and reverse) were used in a PCR reaction composed of 2μl of *Penicillium oxalicum* cDNA, 10 μl of 5X GC Buffer, 1.5μl of DMSO, 2.5 mM each of dATP, dTTP, dGTP, and dCTP, and 0.6 unit of Phusion™ High-Fidelity DNA Polymerase (Finnzymes Oy, Espoo, Finland) in a final volume of 50 μl. The amplification was performed using a Peltier Thermal Cycler (M J Research Inc., South San Francisco, CA, USA) programmed for denaturing at 98°C for 1 minutes; 8 cycles of denaturing at 98°C for 15 seconds, annealing at 65°C for 30 seconds, with 1 °C decrease per cycle and elongation at 72°C for 75 seconds; and another 24 cycles each at 98°C for 15 seconds, 60°C for 30 seconds and 72°C for 75 seconds; final extension at 72°C for 10 minutes. The heat block then went to a 4°C soak cycle.

The PCR products were isolated by 1.0% agarose gel electrophoresis using 90mM Tris-borate and 1 mM EDTA (TBE) buffer where a single product band at 2.0kb of each reaction was visualized under UV light. PCR products were then purified from solution by using an illustra GFX PCR DNA and Gel Band Purification Kit (GE Healthcare, Buckinghamshire, UK) according to the manufacturer's instructions.

Plasmid pLIZG8HQ was digested with *Bam* I and *EcoR* I, isolated by 1.0% agarose gel electrophoresis using TBE buffer (Tris/Borate/EDTA buffer), and purified using an illustra GFX PCR DNA and Gel Band Purification Kit according to the manufacturer's instructions.

An In-fusion CF Dry-down Cloning Kit (Clontech Laboratories, Inc., Mountain View, CA, USA) was used to clone the fragment directly into the expression vector pLIZG8HQ, without the need for restriction digestion and ligation.

The respective PCR products S1 and S2 were ligated to the digested vector pLIZG8HQ using an In-fusion CF Dry-down PCR Cloning kit (Clontech Laboratories, Inc., Mountain View, CA, USA) resulting in plasmids pS1 and pS2 respectively. The cloning operation was according to the manufacturer's instruction. In brief, for each ligation reaction 30 ng of pLIZG8HQ digested with *Bam* I and *EcoR* I, and 60 ng of the *Penicillium oxalicum* alpha-amylase purified PCR product were added to the reaction vial and resuspended the powder in a final volume of 10μl with addition of deionized water. The reaction was incubated at 37°C for 15 minutes and then 50°C for 15 minutes. Three microliters of the reaction products were used to transform *E. coli* TOP10 competent cells (TIANGEN Biotech (Beijing) Co. Ltd., Beijing, China). *E. coli* transformants containing expression constructs were detected by colony PCR and plasmid DNA was prepared using a QIAprep Spin Miniprep Kit (QIAGEN Inc., Valencia, CA, USA). The *Penicillium oxalicum* alpha-amylase genes inserted in pS1 and pS2 were confirmed by DNA sequencing using 3730XL DNA Analyzers (Applied Biosystems Inc, Foster City, CA, USA).

Example 5: Expression of *Penicillium oxalicum* alpha-amylase genes in *Pichia pastoris*

*Pichia pastoris* competent cell preparation

The optical density (OD) of the overnight culture of *Pichia pastoris* in YPM in shaking flask was 1.0. Cells were pelleted by centrifugation at 2000rpm, 5 mins, 4°C. Cell pellet was then suspended in YPD plus 4-(2-hydroxyethyl)-1-piperazineethanesulfonic acid (HEPES) and Dithiothreitol (DTT) and stand at 30°C for 15mins. Cells were pelleted and washed with cold water and 1 M sorbitol subsequenctly. Finally cells were suspended in small amount of 1 M sorbitol and stored in 40μl aliquots at -70°C.

Transformation of pS1 and pS2 to *Pichia pastoris*

Five milligrams of plasmid DNA pS1 or pS2 were linearized with *Pme*I leading to insertion of the plasmid at the chromosomal 5'AOX1 locus. Linearized plasmid DNA (500ng) was mixed with 40μl of competent cells and stored on ice for 5 min. Cells were transferred to an ice-cold 0.2 cm electroporation cuvette. Transformation was performed using a GenePulser II (BioRad, 2000 Alfred Nobel Drive, Hercules, CA 94547). Parameters used were 1500 V, 50μF and 200Ω. Immediately after pulsing, cells were suspended in 1 ml of ice cold 1 M sorbitol. The mixtures were plated on MD plates. Plates were incubated at 28°C for 3-4 days.

Screening clones for expression in small scale

Four candidate clones from each transformation were cultured in a 3 ml scale using 24-deep well plates (Whatman, UK). Cells were grown in BMSY media for 2.5 days at 28°C with vigorous shaking. 0.5% methanol was added to the culture to induce heterologous gene expression. Culture was continuously grown for 4 days with a daily addition of 0.5% methanol under the same growth condition. Samples of culture were taken daily during induction and stored at -20°C for SDS-PAGE analysis and amylase activity assay.

The culture of pS1 and pS2 showed a protein band at 55KD and 65KD respectively visualized on SDS-PAGE and amylase activity by testing against AZCL-amylose.

Example 6: The purification of the alpha-amylases of the present invention

The pH of culture supernatant was adjusted to 7.0 with NaOH, then filtered through a 0.45 μm filter. The solution was applied to a 30ml Ni-sepharose High Performance column (GE Healthcare) equilibrated with 20mM phosphate buffered saline (PBS) containing 0.3 M NaCl at pH7.0. The protein was eluted with a linear imidazole gradient (0-500mM). Fractions from the column were analyzed for amylase activity.

Fractions with amylase activity were checked by SDS-PAGE and the pure fractions were pooled. The SDS-PAGE showed the molecular weight of P243SV was about 55 kDa and the molecular weight of P23WU1 was about 65 kDa.

Example 7: Characterization of the alpha-amylase of the present invention

These two alpha-amylases as purified in example 6 were analysed. The results were shown in table 2 and table 3, respectively.

Table 2 The analytical result of P243SV

| Analysis | Entire Protein |
|---|---|
| Length | 505 aa |
| Molecular Weight | 56742.60 dalton |
| 1 microgram = | 17.623 pMoles |

(continued)

| Analysis | Entire Protein |
|---|---|
| Molar Extinction coefficient | 92660 |
| A[280] of 1 mg/ml | 1.63 AU |
| Isoelectric Point | 5.12 |
| Charge at pH 7 | -13.51 |

Table 3 The analytical result of P23WU1

| Analysis | Entire Protein |
|---|---|
| Length | 619 aa |
| Molecular Weight | 66895.60 |
| 1 microgram = | 14.949 pMoles |
| Molar Extinction coefficient | 128010 |
| A[280] of 1 mg/ml | 1.91 AU |
| Isoelectric Point | 5.62 |
| Charge at pH 7 | -6.67 |

These two alpha-amylases as purified in example 6 were further characterized according to the following methods.

AZCL-HE-amylose Assay

Three microliters of alpha-amylase samples and 100$\mu$l 0.2% AZCL-HE-amylose (Megazyme International Ireland Ltd.) at pH 4.3 were mixed separately in a Microtiter plate and placed on ice before reaction. The assay was initiated by transferring the Microtiter plate to an Eppendorf thermomixer, which was set to the assay temperature 40°C. Then 60$\mu$l supernatant was transferred to a new microtiter plate. Optical Density at 595 nm ($OD_{595}$) was read as a measure of amylase activity. All reaction was done with duplicate and a buffer blind was included in the assay (instead of enzyme).

The adsorbances for P243SV and P23WU1 at $OD_{595}$ is 0.33 and 0.67, respectively, showing P243SV and P23WU1 have amylase activity.

pH Profile

Three microliters of enzyme samples and 40 $\mu$l 1% AZCL-HE-amylose in 100 $\mu$l B&R buffer (Britton-Robinson buffer: 0.1 M boric acid, 0.1 M acetic acid, and 0.1 M phosphoric acid) adjusted to pH-values 3.0, 4.0, 5.0, 6.0, 7.0, 8.0, 9.0, 10.0 and 11.0 with HCl or NaOH were mixed in an Microtiter plate and placed on ice before reaction. The assay was initiated by transferring the Microtiter plate to an Eppendorf thermomixer, which was set to the assay temperature 50°C. Then 60$\mu$l supernatant was transferred to a new microtiter plate. $OD_{595}$ was read as a measure of amylase activity. All reaction was done with duplicate and a buffer blind was included in the assay (instead of enzyme).

Both alpha-amylases are acidic amylases and their optimal pH is pH4.0 as shown in table 4.

Table 4 pH profile of the alpha-amylases

| pH | 2 | 3 | 4 | 5 | 6 | 7 | 8 | 9 | 10 | 11 |
|---|---|---|---|---|---|---|---|---|---|---|
| P243SV | 0.14 | 0.15 | 0.50 | 0.19 | 0.14 | 0.14 | 0.15 | 0.14 | 0.16 | 0.16 |
| P23WU1 | 0.11 | 0.20 | 0.77 | 0.73 | 0.66 | 0.68 | 0.64 | 0.36 | 0.14 | 0.11 |

pH Stability

Twenty microliters of enzyme were added into 100$\mu$l buffer (100mM Na-acetate) at pH4.0, incubated at 37°C for 0, 10, 30, 60 and 120 mins. The enzyme was added into 40 µl 1% AZCL-HE-amylose in water at 37°C for 20min, 60µl taken for $OD_{595}$.

Both alpha-amylases are stable at pH4.0 as shown in table 5.

Table 5 pH stability of the alpha-amylases

| Time (mins) | 0 | 10 | 30 | 60 | 120 |
|---|---|---|---|---|---|
| P243SV | 0.56 | 0.85 | 0.67 | 0.63 | 0.60 |
| P23WU1 | 1.34 | 1.54 | 1.34 | 1.46 | 1.47 |

Temperature profile

Three microliters of enzyme was added into 100µl buffer (50mM NaAc) at pH 4.3 containing 0.2% AZCL-HE-amylose, incubating for 20 mins and 60µl supernatant was taken for $OD_{595}$.

P23WU1 shows higher optimal temperature (60 °C) than P243SV (40 °C) as shown in table 6.

Table 6 Temperature profile of the alpha-amylases

| Temperature (°C) | 30 | 40 | 50 | 60 | 70 | 80 | 90 |
|---|---|---|---|---|---|---|---|
| P243SV | 0.30 | 0.33 | 0.14 | 0.10 | 0.10 | 0.08 | 0.09 |
| P23WU1 | 0.57 | 0.67 | 0.81 | 0.86 | 0.21 | 0.15 | 0.14 |

Temperature stability

Three microliters of enzyme samples were added into 100µl 50 mM NaAc at pH4.3 and incubated at 50 °C (P243SV) and 65 °C (P23WU1) for 0,10,30,60 and 120mins, and then they were put on ice at each time point. Forty microliters of 1% AZCL-HE-amylose in water was added at 37 °C for 20mins, 60µl taken for $OD_{595}$.

P243SV is more stable at 50 °C, and P23WU1 is relatively stable at high temperature (65 °C), as shown in table 7.

Table 7 Temperature stabiltiy of the alpha-amylases

| Time (mins) | 0 | 10 | 30 | 60 | 120 |
|---|---|---|---|---|---|
| P243SV (50 °C) | 0.86 | 0.82 | 0.78 | 0.75 | 0.45 |
| P23WU1 (65 °C) | 0.74 | 0.58 | 0.42 | 0.27 | 0.13 |

Example 8: Simultaneous Saccharification and Fermentation (SSF) performance of recombinant *Penicillium oxalicum* alpha-amylase

The SSF performance of purified *Penicillium oxalicum* alpha-amylase (P243SV or P23WU1) was tested at different enzyme doses in combination with a constant dose (equivalent to 0.56 AGU/gDS) of purified *Talaromyces emersonii* glucoamylase (disclosed in international publication WO 99/28448 as SEQ ID NO: 7) or in combination with a blend of *Talaromyces* emersonii glucoamylase and *Trametes Cingulata* glucoamylase (disclosed in international publication WO 2006/069289 as SEQ ID NO:2). *Talaromyces emersonii* glucoamylase and *Trametes Cingulata* glucoamylase were both expressed in *Aspergillus Niger.*

Fermentation was run under the following conditions:

Substrate: Industrially liquefied corn mash 33.9% DS (%w/w)
Temperature: 32°C
Initial pH: 5.0
Alpha-amylase dose: *Penicillium oxalicum* alpha-amylase (obtained by Example 6) at 3 and 10 µg Enzyme Protein/g Dry Solid (µg EP/g DS).

Experiment A, *Penicillium oxalicum alpha-amylases* were compared to a purified sample of the *Talaromyces emersonii* glucoamylase dosed at same dosages. The dose of *Talaromyces emersonii* glucoamylase is equivalent to an industry relevant 0.56 AGU/gDS.

Experiment B, *Penicillium oxalicum* alpha-amylases were compared to a blend of commercial *Talaromyces emersonii* glucoamylase and *Trametes Cingulata* glucoamylase. The total glucoamylase dose is equivalent to an industry relevant 0.56 AGU/gDS.

Fermentation

The substrate for SSF was sampled after the liquefaction step in an ethanol facility. The ground corn was slurried with backset and other recycled water and adjusted its dry substance to approximately 33.9% (w/w), and it was then sent into liquefaction at 85°C and pH 5.8. Before lab fermentations, 1000 ppm urea as nitrogen source and 3 ppm penicillin for bacterial control were added; the pH was then adjusted to 5.0 with $H_2SO_4$. Aliquots of substrate corresponding to about 5g mash were transferred to 15 ml centrifuge tubes - with a hole drilled at the top for $CO_2$ release. Enzymes and yeast (30 million cells/ml) were added and the tubes were placed in a water bath without stirring at 32°C. Samples were analyzed in High Performance Liquid Chromatography (HPLC) for determination of the carbohydrate and ethanol concentrations.

The ethanol produced during fermentation is given in tables 8 and 9.

Table 8 Ethanol (g/L) produced during SSF with added *Penicillium oxalicum* alpha-amylase (P243SV or P23WU1) was tested at different enzyme doses in combination with *Talaromyces emersonii* glucoamylase) at 0.56 AGU/gDS as compared to *Talaromyces emersonii* glucoamylase alone, Experiment A

| | | Enzyme dose (μgEP/gDS) | |
|---|---|---|---|
| | | 3 | 10 |
| No alpha-amylase | 126.3 | | |
| *Penicillium oxalicum* - P243SV | | 125.6 | 126.0 |
| *Penicillium oxalicum* - P23WU1 | | 129.0 | 129.8 |

Table 9 Ethanol (g/L) produced during SSF with added *Penicillium oxalicum* alpha-amylase (P23WU1) was tested at different enzyme doses in combination with a blend of *Talaromyces emersonii* glucoamylase and *Trametes Cingulata* glucoamylase at 0.56 AGU/gDS as compared to the glucoamylase blend alone, Experiment B

| | | Enzyme dose (μgEP/gDS) | |
|---|---|---|---|
| | | 3 | 10 |
| No alpha-amylase | 120.8 | | |
| *Penicillium oxalicum* - P23WU1 | | 122.6 | 123.6 |

Example 9: the effect of the *Penicillium oxalicum* alpha-amylase on the volume of the bread

Mini rolls were made of 12 g Kolibri® flour (Meneba, Rotterdam, the Netherlands) according to a straight dough procedure with the addition of 56% water, 4.5% yeast, 1.5% sucrose, 1.5% salt and 40 ppm ascorbic acid based on the flour. Dough was supplemented with different dosages of enzymes according to table 10 below. All ingredients were mixed in a mini mixer (NS1-33R, National MFG Co., Lincoln, Nebraska, United states) for 3.5 min at speed 3. After mixing the dough was shaped and placed in a round aluminum form (diameter 90 mm and side height 15 mm). After 55 min proofing at 32°C and 86% Relative humidity (RH), the dough was baked at 230°C for 17 min.

Table 10 Enzyme dosages

| | 1 | 2 | 3 | 4 | 5 | 6 | 7 |
|---|---|---|---|---|---|---|---|
| P23WU1 (mg enzyme protein/kg flour) | | 0.33 | 1 | 3 | | | |
| P243SV (mg enzyme protein/kg flour) | | | | | 0.33 | 1 | 3 |

The effect of the enzyme on the volume of the breads was evaluated by a water displacement method, i.e., measuring the amount of water displaced by the bread at room temperature. A beaker containing water was put on a balance, which was then adjusted to 0.000 g. The bread was impregnated with paraffin at 80 °C for 3 second, allowed to cool for 10 second, and then immersed into the water in the beaker by pressure. The mass of the displaced water (gram), or the pressure applied, was read from the balance (gram). The mass of the displaced water corresponded to the volume of the bread (ml).

The bread was weighted on a balance and the specific volume was calculated by dividing the volume of bread (from water displacement method)) by the weight of bread (determined by weighing bread). A specific volume index was calculated by setting the specific volume of the control bread to 100% and calculating the specific volume of the enzyme treated bread relative to the control (no enzyme).

The effect of amylase P23WU1 and amylase P243SV on volume of mini rolls can be seen in table 2 below.

Table 2 Volume of rolls with different amylases

| Enzyme | specific volume (ml/g) | specific volume index (%) |
|---|---|---|
| Control (no enzyme) | 2.993 | 100 |
| P23WU1 (0.33 mg/kg) | 3.138 | 105 |
| P23WU1 (1 mg/kg) | 3.055 | 102 |
| P23WU1 (3 mg/kg) | 3.107 | 104 |
| P243SV (0.33 mg/kg) | 3.036 | 101 |
| P243SV (1 mg/kg) | 3.035 | 101 |
| P243SV (3 mg/kg) | 2.924 | 98 |

The amylase P23WU1 was able to improve the volume of the bread at 0.33 mg enzyme protein/kg flour, 1 mg enzyme protein/kg flour, and 3mg enzyme protein/kg flour, respectively. The amylase P243SV was able to improve the volume of the bread at 0.33 mg enzyme protein/kg flour and 1 mg enzyme protein/kg flour, respectively.

The present invention is further described by the following numbered paragraphs:

[1] An isolated polypeptide having alpha-amylase activity, selected from the group consisting of:

(a) a polypeptide having at least 80%, e.g., at least 85%, at least 90%, at least 91 %, at least 92%, at least 93%, at least 94%, at least 95%, at least 96%, at least 97%, at least 98%, at least 99%, or 100% sequence identity to the mature polypeptide of SEQ ID NO: 4; or a polypeptide having at least 65%, e.g., at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the mature polypeptide of SEQ ID NO:2;

(b) a polypeptide encoded by a polynucleotide that hybridizes under low stringency conditions, medium stringency conditions, medium-high stringency conditions, high stringency conditions, or very high stringency conditions with (i) the mature polypeptide coding sequence of SEQ ID NO: 3, or the mature polypeptide coding sequence of SEQ ID NO: 1, (ii) the cDNA sequences thereof, or (iii) the full-length complement of (i) or (ii);

(c) a polypeptide encoded by a polynucleotide having at least 80%, e.g., at least 85%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 3 or the cDNA sequence thereof; or a polypeptide encoded by a polynucleotide having at least 65%, e.g., at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 1 or the cDNA sequence thereof;

(d) a variant of the mature polypeptide of SEQ ID NO: 4 or the mature polypeptide of SEQ ID NO: 2 comprising a substitution, deletion, and/or insertion at one or more positions; and

(e) a fragment of the polypeptide of (a), (b), (c), or (d) that has alpha-amylase activity.

[2] The polypeptide of paragraph 1, having at least 80%, at least 85%, at least 90%, at least 91%, at least 92%, at least 93%, at least 94%, at least 95%, at least 96%, at least 97%, at least 98%, at least 99%, or 100% sequence identity to the mature polypeptide of SEQ ID NO: 4; or at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the mature polypeptide of SEQ ID NO: 2.

[3] The polypeptide of paragraph 1 or 2, which is encoded by a polynucleotide that hybridizes under low stringency conditions, low-medium stringency conditions, medium stringency conditions, medium-high stringency conditions, high stringency conditions, or very high stringency conditions with (i) the mature polypeptide coding sequence of SEQ ID NO: 3 or the mature polypeptide coding sequence of SEQ ID NO: 1, (ii) the cDNA sequence thereof, or (iii) the full-length complement of (i) or (ii).
[4] The polypeptide of any of paragraphs 1-3, which is encoded by a polynucleotide having at least 80%, at least 85%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 3 or the cDNA sequence thereof; or which is encoded by a polynucleotide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 1 or the cDNA sequence thereof.
[5] The polypeptide of any of paragraphs 1-4, comprising or consisting of SEQ ID NO: 4, or SEQ ID NO: 2, the mature polypeptide of SEQ ID NO: 4, or the mature polypeptide of SEQ ID NO: 2.
[6] The polypeptide of paragraph 5, wherein the mature polypeptide is amino acids 21 to 619 of SEQ ID NO: 4, or amino acids 26 to 505 of SEQ ID NO: 2.
[7] The polypeptide of any of paragraphs 1-4, which is a variant of the mature polypeptide of SEQ ID NO: 4 or the mature polypeptide of SEQ ID NO: 2, comprising a substitution, deletion, and/or insertion at one or more positions; or which is a fragment of SEQ ID NO: 2 or SEQ ID NO: 4, wherein the fragment has alpha-amylase activity.
[8] The polypeptide of any of paragraphs 1-7, which is stable at 10 °C-100 °C, preferably 20 °C- 80 °C, more preferably 30 °C- 70 °C.
[9] The polypeptide of any of paragraphs 1-8, which is a fragment of the polypeptide of SEQ ID NO: 4, or SEQ ID NO: 2, having alpha-amylase activity.
[10] An isolated polypeptide comprising a catalytic domain selected from the group consisting of:

(a) a catalytic domain having at least 80% sequence identity to amino acids 21 to 493 of SEQ ID NO: 4, or at least 65% sequence identity to amino acids 26 to 503 of SEQ ID NO: 2;
(b) a catalytic domain encoded by a polynucleotide that hybridizes under low, medium, medium-high, high, or very high stringency conditions with nucleotides 61 to 1973 of SEQ ID NO: 3, or nucleotides 76 to 1578 of SEQ ID NO: 1, (ii) the cDNA sequence thereof,or (iii) the full-length complement of (i) or (ii);
(c) a catalytic domain encoded by a polynucleotide having at least 80% sequence identity to nucleotides 61 to 1973 of SEQ ID NO: 3, or at least 65% sequence identity to nucleotides 76 to 1578 of SEQ ID NO: 1;
(d) a variant of amino acids 21 to 493 of SEQ ID NO: 4 or amino acids 26 to 503 of SEQ ID NO: 2, comprising a substitution, deletion, and/or insertion at one or more positions; and
(e) a fragment of the catalytic domain of (a), (b), (c), or (d) that has alpha-amylase activity.

[11] The polypeptide of paragraph 10, further comprising a carbohydrate binding domain.
[12] An isolated polypeptide comprising a carbohydrate binding domain, wherein the binding domain is selected from the group consisting of:

(a) a carbohydrate binding domain having at least 80% sequence identity to amino acids 511 to 619 of SEQ ID NO: 4;
(b) a carbohydrate binding domain encoded by a polynucleotide that hybridizes under low, medium, medium-high, high, or very high stringency conditions with (i) nucleotides 2025 to 2351 of SEQ ID NO: 3, (ii) the cDNA sequence thereof, or (iii) the full-length complement of (i) or (ii);
(c) a carbohydrate binding domain encoded by a polynucleotide having at least 80% sequence identity to nucleotides 2025 to 2351 of SEQ ID NO: 3 or the cDNA sequence thereof;
(d) a variant of amino acids 511 to 619 of SEQ ID NO: 4 comprising a substitution, deletion, and/or insertion at one or more positions; and
(e) a fragment of (a), (b), (c), (d) or (e) that has carbohydrate binding activity.

[13] The polypeptide of paragraph 12, wherein the carbohydrate binding domain is operably linked to a catalytic domain.
[14] The polypeptide of paragraph 13, wherein the catalytic domain is obtained from amylase.
[15] The polypeptide of paragraph 14, wherein the catalytic domain is obtained from alpha-amylase.
[16] The polypeptide of paragraph 15, wherein the catalytic domain is obtained from acid alpha-amylase.
[17] A whole broth formulation or cell culture composition comprising the polypeptide of any of paragraphs 1-16.
[18] A composition comprising the polypeptide of any of paragraphs 1-16 and an enzyme selected from the group consisting of: a fungal alpha-amylase (EC 3.2.1.1), a beta-amylase (E.C. 3.2.1.2), a glucoamylase (E.C.3.2.1.3), a pullulanases (E.C. 3.2.1.41), a phytase (E.C.3.1.2.28) and a protease (E.C. 3.4.).

[19] An isolated polynucleotide encoding the polypeptide of any of paragraphs 1-16.

[20] A nucleic acid construct or expression vector comprising the polynucleotide of paragraph 19 operably linked to one or more control sequences that direct the production of the polypeptide in an expression host.

[21] A recombinant host cell comprising the polynucleotide of paragraph 19 operably linked to one or more control sequences that direct the production of the polypeptide.

[22] A method of producing the polypeptide of any of paragraphs 1-16, comprising:

cultivating a cell, which in its wild-type form produces the polypeptide, under conditions conducive for production of the polypeptide.

[23] A method of paragraph 22, further comprising recovering the polypeptide.

[24] A method of producing a polypeptide having alpha-amylase activity, comprising:

cultivating the host cell of paragraph 21 under conditions conducive for production of the polypeptide.

[25] A method of paragraph 24, further comprising recovering the polypeptide.

[26] A transgenic plant, plant part or plant cell transformed with a polynucleotide encoding the polypeptide of any of paragraphs 1-16.

[27] A method of producing a polypeptide having alpha-amylase activity, comprising:

cultivating the transgenic plant or plant cell of paragraph 26 under conditions conducive for production of the polypeptide.

[28] A method of paragraph 27, further comprising recovering the polypeptide.

[29] A method of producing a mutant of a parent cell, comprising inactivating a polynucleotide encoding the polypeptide of any of paragraphs 1-16, which results in the mutant producing less of the polypeptide than the parent cell.

[30] A mutant cell produced by the method of paragraph 29.

[31] The mutant cell of paragraph 30, further comprising a gene encoding a native or heterologous protein.

[32] A method of producing a protein, comprising:

cultivating the mutant cell of paragraph 30 or 31 under conditions conducive for production of the protein.

[33] A method of paragraph 32, further comprising recovering the polypeptide.

[34] A double-stranded inhibitory RNA (dsRNA) molecule comprising a subsequence of the polynucleotide of paragraph 19, wherein optionally the dsRNA is an siRNA or an miRNA molecule.

[35] The double-stranded inhibitory RNA (dsRNA) molecule of paragraph 34, which is about 15,16,17,18,19,20,21,22,23,24,25 or more duplex nucleotides in length.

[36] A method of inhibiting the expression of a polypeptide having alpha-amylase activity in a cell, comprising administering to the cell or expressing in the cell the double-stranded inhibitory RNA (dsRNA) molecule of paragraph 34 or 35.

[37] A cell produced by the method of paragraph 36.

[38] The cell of paragraph 37, further comprising a gene encoding a native or heterologous protein.

[39] A method of producing a protein, comprising:

cultivating the cell of paragraph 37 or 38 under conditions conducive for production of the protein.

[40] A method of paragraph 39, further comprising recovering the polypeptide.

[41] An isolated polynucleotide encoding a signal peptide comprising or consisting of amino acids 1 to 20 of SEQ ID NO: 4, or amino acids 1 to 25 of SEQ ID NO: 2.

[44] A nucleic acid construct or expression vector comprising a gene encoding a protein operably linked to the polynucleotide of paragraph 41, wherein the gene is foreign to the polynucleotide encoding the signal peptide.

[45] A recombinant host cell comprising a gene encoding a protein operably linked to the polynucleotide of paragraph 41, wherein the gene is foreign to the polynucleotide encoding the signal peptide.

[46] A method of producing a protein, comprising:

cultivating a recombinant host cell comprising a gene encoding a protein operably linked to the polynucleotide of paragraph 41, wherein the gene is foreign to the polynucleotide encoding the signal peptide, under conditions conducive for production of the protein.

[47] A method of paragraph 46, further comprising recovering the protein.

[48] Use of the polypeptide according to any of paragraphs 1-16, the whole broth formulation or cell culture composition according to paragraph 17 or the composition according to paragraph 18 for starch conversion.

[49] Use of the polypeptide according to any of paragraphs 1-16, the whole broth formulation or cell culture composition according to paragraph 17 or the composition according to paragraph 18 for starch modification in the paper and pulp industry.

[50] Use of the polypeptide according to any of paragraphs 1-16, the whole broth formulation or cell culture composition according to paragraph 17 or the composition according to paragraph 18 for starch liquefaction and/or saccharification.

[51] Use of the polypeptide according to any of paragraphs 1-16, the whole broth formulation or cell culture composition according to paragraph 17 or the composition according to paragraph 18 for textile washing.

[52] Use of the polypeptide according to any of paragraphs 1-16, the whole broth formulation or cell culture composition according to paragraph 17 or the composition according to paragraph 18 for textile desizing.

[53] Use of the polypeptide according to any of paragraphs 1-16, the whole broth formulation or cell culture composition according to paragraph 17 or the composition according to paragraph 18 for brewing.

[54] Use of the polypeptide according to any of paragraphs 1-16, the whole broth formulation or cell culture composition according to paragraph 17 or the composition according to paragraph 18 for ethanol production.

[55] Use according to paragraph 54 for production of ethanol in a process comprising hydrolyzing starch.

[56] Use of the polypeptide according to any of paragraphs 1-16, the whole broth formulation or cell culture composition according to paragraph 17 or the composition according to paragraph 18 for baking.

[57] Use according to paragraph 56 for the improvement of the volume of a bread.

[58] A method for converting starch, preferably gelatinized starch, wherein a polypeptide according to any of paragraphs 1-16, the whole broth formulation or cell culture composition according to paragraph 17 or the composition according to paragraph 18 is added.

[59] A method for modificating starch in the paper and pulp industry, wherein a polypeptide according to any of paragraphs 1-16, the whole broth formulation or cell culture composition according to paragraph 17 or the composition according to paragraph 18 is added.

[60] A method for liquefying and/or saccharifying starch, wherein a polypeptide according to any of paragraphs 1-16, the whole broth formulation or cell culture composition according to paragraph 17 or the composition according to paragraph 18 is added.

[61] A method for washing textile, wherein a polypeptide according to any of paragraphs 1-16, the whole broth formulation or cell culture composition according to paragraph 17 or the composition according to paragraph 18 is added.

[62] A method for desizing textile, wherein a polypeptide according to any of paragraphs 1-16, the whole broth formulation or cell culture composition according to paragraph 17 or the composition according to paragraph 18 is added.

[63] A method for brewing, wherein a polypeptide according to any of paragraphs 1-16, the whole broth formulation or cell culture composition according to paragraph 17 or the composition according to paragraph 18 is added.

[64] A method for producing ethanol, wherein a polypeptide according to any of paragraphs 1-16, the whole broth formulation or cell culture composition according to paragraph 17 or the composition according to paragraph 18 is added.

[65] A method according to paragraph 64, wherein the ethanol is produced in a process comprising hydrolyzing starch.

[66] A method for baking, wherein a polypeptide according to any of paragraphs 1-16, the whole broth formulation or cell culture composition according to paragraph 17 or the composition according to paragraph 18 is added.

[67] A method according to paragraph 66, wherein a polypeptide according to any of paragraphs 1-16, the whole broth formulation or cell culture composition according to paragraph 17 or the composition according to paragraph 18 is added to dough.

## Claims

1. An isolated polypeptide having alpha-amylase activity, selected from the group consisting of:

   a polypeptide having at least 90%, at least 91%, at least 92%, at least 93%, at least 94%, at least 95%, at least 96%, at least 97%, at least 98%, at least 99%, or 100% sequence identity to amino acids 21-619 of SEQ ID NO: 4; wherein % identity is determined using the Needle program of the EMBOSS package with a gap open penalty of 10, a gap extension penalty of 0.5, and the EBLOSUM62 by the calculation: (Identical Residues x 100)/(Length of Alignment - Total Number of Gaps in Alignment).

2. The polypeptide of claim 1, comprising or consisting of SEQ ID NO: 4, or amino acids 21-619 of SEQ ID NO: 4.

3. An isolated polypeptide comprising a catalytic domain having at least 95% sequence identity to amino acids 21 to 493 of SEQ ID NO: 4.

4. An isolated polynucleotide encoding the polypeptide of any of claims 1-3.

5. A method of producing the polypeptide of any of claims 1-3, comprising:

   (a) cultivating a cell, which in its wild-type form produces the polypeptide, under conditions conducive for production of the polypeptide; and
   (b) recovering the polypeptide.

6. A method of producing a polypeptide having alpha-amylase activity, comprising:

   (a) cultivating a recombinant host cell comprising the polynucleotide of claim 4 under conditions conducive for production of the polypeptide; and optionally
   (b) recovering the polypeptide.

7. A whole broth formulation or cell culture composition comprising the polypeptide of any of claims 1-3.

8. A composition comprising the polypeptide of any of claims 1-3 and an enzyme selected from the group consisting of: a fungal alpha-amylase EC 3.2.1.1, a beta-amylase EC 3.2.1.2, a glucoamylase EC 3.2.1.3, a pullulanases EC 3.2.1.41, a phytase EC 3.1.2.28, and a protease EC 3.4..

9. Use of the polypeptide according to any of claims 1-3, the whole broth formulation or cell culture composition according to claim 7 or the composition according to claim 8 for starch conversion, starch modification in the paper and pulp industry, starch liquefaction and/or saccharification, textile washing, textile desizing, brewing, ethanol production and/or baking.

10. A method for converting starch, modificating starch in the paper and pulp industry, liquefying and/or saccharifying starch, washing textile, desizing textile, brewing, producing ethanol and/or baking, wherein a polypeptide according to any of claims 1-3 is added.

11. A recombinant host cell comprising the polynucleotide of claim 4.

12. The recombinant host cell according to claim 11, wherein the host cell is a yeast cell.

13. The recombinant host cell according to claim 12, wherein the yeast is a *Saccharomyces cerevisiae.*

## Patentansprüche

1. Isoliertes Polypeptid mit alpha-Amylaseaktivität, ausgewählt aus der Gruppe bestehend aus:

   einem Polypeptid mit mindestens 90%, mindestens 91%, mindestens 92%,
   mindestens 93%, mindestens 94%, mindestens 95%, mindestens 96%, mindestens 97%, mindestens 98%, mindestens 99%, oder 100% Sequenzidentität zu Aminosäuren 21-619 von SEQ ID NO: 4; wobei % Identität unter Verwendung des Needle-Programms des EMBOSS-Pakets mit einem Lückenöffnungsstrafwert von 10, einem Lückenerweiterungsstrafwert von 0,5 und der EBLOSUM62 durch die Berechnung: (Identische Reste x 100)/(Länge des Abgleichs - Gesamtzahl an Lücken im Abgleich) bestimmt ist.

**2.** Polypeptid nach Anspruch 1, umfassend oder bestehend aus SEQ ID NO: 4, oder Aminosäuren 21-619 von SEQ ID NO: 4.

**3.** Isoliertes Polypeptid, umfassend eine katalytische Domäne mit mindestens 95% Sequenzidentität zu Aminosäuren 21 bis 493 von SEQ ID NO: 4.

**4.** Isoliertes Polynukleotid, das das Polypeptid gemäß einem beliebigen der Ansprüche 1-3 kodiert.

**5.** Verfahren zum Herstellen des Polypeptids gemäß einem beliebigen der Ansprüche 1-3, umfassend:

(a) Kultivieren einer Zelle, die in ihrer Wildtyp-Form das Polypeptid herstellt, unter Bedingungen, die für die Herstellung des Polypeptids förderlich sind; und
(b) Gewinnen des Polypeptids.

**6.** Verfahren zum Herstellen eines Polypeptids mit alpha-Amylaseaktivität, umfassend:

(a) Kultivieren einer rekombinanten Wirtszelle, die das Polynukleotid gemäß Anspruch 4 umfasst, unter Bedingungen, die für die Herstellung des Polypeptids förderlich sind; und gegebenenfalls
(b) Gewinnen des Polypeptids

**7.** Gesamtbrühenformulierung oder Zellkulturzusammensetzung, umfassend das Polypeptid gemäß einem beliebigen der Ansprüche 1-3.

**8.** Zusammensetzung, umfassend das Polypeptid gemäß einem beliebigen der Ansprüche 1-3 und ein Enzym, ausgewählt aus der Gruppe bestehend aus: einer pilzlichen alpha-Amylase EC 3.2.1.1, einer beta-Amylase E.C. 3.2.1.2, einer Glucoamylase E.C. 3.2.1.3, einer Pullulanase E.C. 3.2.1.41, einer Phytase E.C. 3.1.2.28 und einer Protease E.C. 3.4.

**9.** Verwendung des Polypeptids gemäß einem beliebigen der Ansprüche 1-3, der Gesamtbrühenformulierung oder Zellkulturzusammensetzung gemäß Anspruch 7, oder der Zusammensetzung gemäß Anspruch 8 zur Stärkeumwandlung, Stärkemodifikation in der Papier- und Pulpeindustrie, Stärkeverflüssigung und/oder Verzuckerung, zum Textilwaschen, Textilentschlichten, Brauen, zur Ethanolherstellung und/oder zum Backen.

**10.** Verfahren zum Umwandeln von Stärke, Modifizieren von Stärke in der Papier- und Pulpeindustrie, Verflüssigen und/oder Verzuckern von Stärke, Waschen von Textil, Entschlichten von Textil, Brauen, Herstellen von Ethanol und/oder Backen, wobei ein Polypeptid gemäß einem beliebigen der Ansprüche 1-3 zugegeben wird.

**11.** Rekombinante Wirtszelle, umfassend das Polynukleotid gemäß Anspruch 4.

**12.** Rekombinante Wirtszelle nach Anspruch 11, wobei die Wirtszelle eine Hefezelle ist.

**13.** Rekombinante Wirtszelle nach Anspruch 12, wobei die Hefe eine *Saccharomyces cerevisiae* ist.

## Revendications

**1.** Polypeptide isolé ayant une activité alpha-amylase, sélectionné dans le groupe consistant en :

un polypeptide ayant une identité de séquence d’au moins 90 %, d’au moins 91 %, d’au moins 92 %, d’au moins 93 %, d’au moins 94 %, d’au moins 95 %, d’au moins 96 %, d’au moins 97 %, d’au moins 98 %, d’au moins 99 %, voire de 100 % avec les acides aminés 21-619 de SEQ ID NO : 4 ; dans lequel le % d’identité est déterminé à en utilisant le programme Needle du progiciel EMBOSS avec une pénalité d’ouverture de brèche de 10, une pénalité d’extension de brèche de 0,5, et EBLOSUM62 par le calcul :

(résidus identiques x 100)/(longueur d'alignement – nombre total de brèches dans l'alignement).

**2.** Polypeptide selon la revendication 1, comprenant ou consistant en SEQ ID NO : 4, ou les acides aminés 21-619 de SEQ ID NO : 4.

**3.** Polypeptide isolé comprenant un domaine catalytique présentant une identité de séquence d'au moins 95 % avec les acides aminés 21 à 493 de SEQ ID NO : 4.

**4.** Polynucléotide isolé codant pour le polypeptide selon l'une quelconque des revendications 1 à 3.

**5.** Méthode de production du polypeptide selon l'une quelconque des revendications 1 à 3, comprenant :

(a) la culture d'une cellule, qui sous sa forme de type sauvage produit le polypeptide, dans des conditions aptes à induire la production du polypeptide ; et
(b) la récupération du polypeptide.

**6.** Méthode de production d'un polypeptide ayant une activité alpha-amylase, comprenant :

(a) la culture d'une cellule hôte recombinante comprenant le polynucléotide selon la revendication 4 dans des conditions aptes à induire la production du polypeptide ; et optionnellement
(b) la récupération du polypeptide.

**7.** Formulation de bouillon entier ou composition de culture cellulaire comprenant le polypeptide selon l'une quelconque des revendications 1 à 3.

**8.** Composition comprenant le polypeptide selon l'une quelconque des revendications 1 à 3 et une enzyme sélectionnée dans le groupe consistant en : une alpha-amylase fongique EC 3.2.1.1, une bêta-amylase EC 3.2.1.2, une glucoamylase EC 3.2.1.3, une pullulanase EC 3.2.1.41, une phytase EC 3.1.2.28, et une protéase EC 3.4.

**9.** Utilisation du polypeptide selon l'une quelconque des revendications 1 à 3, de la formulation de bouillon entier ou de la composition de culture cellulaire selon la revendication 7 ou de la composition selon la revendication 8 pour la conversion de l'amidon, la modification de l'amidon dans l'industrie du papier et de la pâte à papier, la liquéfaction et/ou la saccharification de l'amidon, le lavage des textiles, le désencollage des textiles, le brassage, la production d'éthanol et/ou la cuisson.

**10.** Méthode de conversion de l'amidon, modification de l'amidon dans l'industrie du papier et de la pâte à papier, liquéfaction et/ou saccharification de l'amidon, lavage des textiles, désencollage des textiles, brassage, production d'éthanol et/ou cuisson, dans laquelle un polypeptide selon l'une quelconque des revendications 1 à 3 est ajouté.

**11.** Cellule hôte recombinante comprenant le polynucléotide selon la revendication 4.

**12.** Cellule hôte recombinante selon la revendication 11, dans laquelle la cellule hôte est une cellule de levure.

**13.** Cellule hôte recombinée selon la revendication 12, dans laquelle la levure est une *Saccharomyces cerevisiae.*

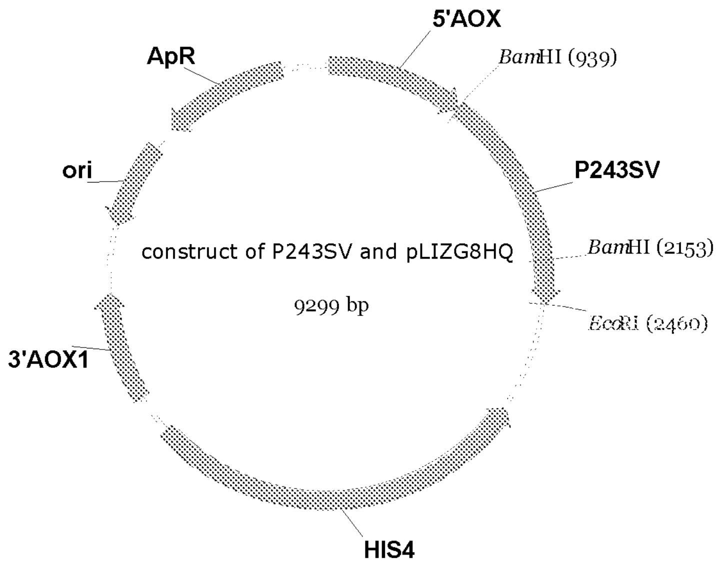
5'AOX
BamHI (939)
P243SV
BamHI (2153)
EcoRI (2460)
HIS4
3'AOX1
ori
ApR
construct of P243SV and pLIZG8HQ
9299 bp

Fig 1

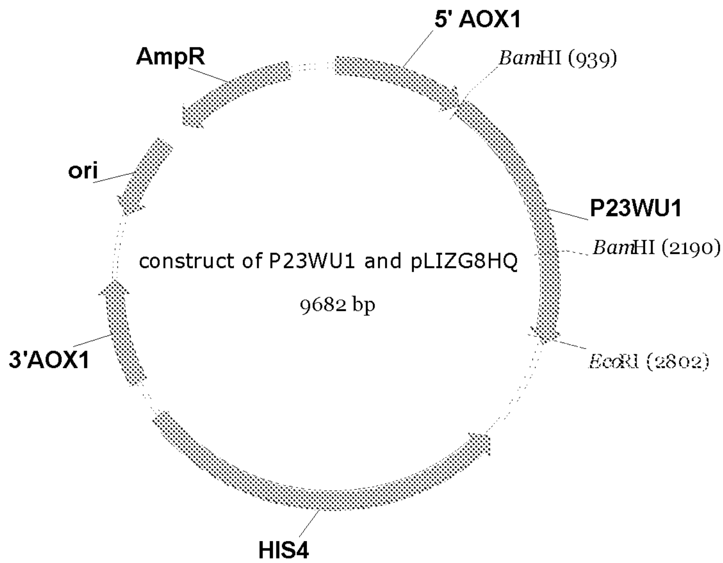
5' AOX1
BamHI (939)
P23WU1
BamHI (2190)
EcoRI (2802)
HIS4
3'AOX1
ori
AmpR
construct of P23WU1 and pLIZG8HQ
9682 bp

Fig 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003012071 A
- WO 2010091221 A
- WO 9517413 A
- WO 9522625 A
- US 5223409 A
- WO 9206204 A
- WO 9943835 A
- WO 9600787 A
- WO 0056900 A
- US 6011147 A
- WO 9425612 A
- WO 9533836 A
- WO 2010039889 A
- WO 0024883 A
- EP 238023 A
- US RE32153 E
- US 4587215 A
- WO 9928448 A
- WO 9015861 A
- WO 2010096673 A
- WO 2006069289 A


### Non-patent literature cited in the description


- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **SAMBROOK et al.** Molecular Cloning, A Laboratory Manual. Cold Spring Harbor, 1989
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **INNIS et al.** PCR: A Guide to Methods and Application. Academic Press, 1990
- **FORD et al.** *Protein Expression and Purification,* 1991, vol. 2, 95-107
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** Useful proteins from recombinant bacteria. *Scientific American,* vol. 242, 74-94
- **ROMANOS et al.** *Yeast,* 1992, vol. 8, 423-488

- **HUE et al.** *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev.,* 1981, vol. 45, 409-436
- Biology and Activities of Yeast. Soc. App. Bacteriol. Symposium Series
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- **BECKER ; GUARENTE.** Guide to Yeast Genetics and Molecular Biology, Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989
- **LI et al.** De novo assembly of human genomes with massively parallel short read sequencing. *Genome Res,* 2010, vol. 20 (2), 265-72
- **PARRA et al.** *Genome Research,* 2000, vol. 10 (4), 511-515
- **ALTSCHUL SF ; GISH W ; MILLER W ; MYERS EW ; LIPMAN DJ.** Basic local alignment search tool. *J Mol Biol,* October 1990, vol. 215 (3), 403-410, http://blast.ncbi.nlm.nih.gov/Blast.cgi
- **MUNCH K ; KROGH A.** Automatic generation of gene finders for eukaryotic species. *BMC Bioinformatics,* 2006, vol. 7, 263
- **RICE P ; LONGDEN I ; BLEASBY A.** EMBOSS: The European Molecular Biology Open Software Suite. *Trends Genet.,* 2000, vol. 16 (6), 276-277